# EUROPEAN PATENT SPECIFICATION

(11) **EP 2 800 778 B1**
(45) Date of publication and mention of the grant of the patent: **22.08.2018**
(21) Application number: 13700597.1
(22) Date of filing: 04.01.2013
(51) Int. Cl.: C08G 77/458, C08G 18/50, C08G 18/61, C08K 5/00, C12Q 1/00, C08K 5/13, C08G 18/28, C08G 18/32, C08G 18/75

(54) **STABILIZED POLYMERS FOR USE WITH ANALYTE SENSORS AND METHODS FOR MAKING AND USING THEM**
STABILISIERTE POLYMERE ZUR VERWENDUNG MIT ANALYTSENSOREN SOWIE VERFAHREN ZU IHRER HERSTELLUNG UND VERWENDUNG
POLYMÈRES STABILISÉS POUR UNE UTILISATION AVEC DES DÉTECTEURS D'ANALYTES ET LEURS PROCÉDÉS DE FABRICATION ET D'UTILISATION

(30) Priority: 05.01.2012 US 201213344343
(43) Date of publication of application: 12.11.2014
(73) Proprietor: Medtronic MiniMed, Inc., Northridge, CA 91325-1219 (US)
(72) Inventor: WANG, Jenn-Hann, L., Northridge, CA 91326 (US); COCHRAN, Brooks, B., Northridge, CA91326 (US); DANG, Tri, T., Winnetka, CA 91306 (US); SHAH, Rajiv, Rancho Palos Verdes, CA 90275 (US)
(74) Representative: Ruschke, Hans Edvard
(86) International application number: PCT/US2013/020229
(87) International publication number: WO 2013/103772

(56) References cited:
- DE-A1-102007 027 027
- DE-A1-102009 046 850
- US-A1- 2006 063 894
- US-B2- 6 642 015

## Description

This application claims the benefit of United States Utility Patent Application Number 13/344,343 filed January 5, 2012.

### BACKGROUND OF THE INVENTION

### 1. Field of the Invention.

This invention relates to biosensors such as glucose sensors used in the management of diabetes and in particular materials used to make such sensors.

### 2. Description of Related Art.

Analyte sensors such as biosensors include devices that use biological elements to convert a chemical analyte in a matrix into a detectable signal. There are many types of biosensors used to detect wide variety of analytes. Perhaps the most studied type of biosensor is the amperometric glucose sensor, an apparatus commonly used to monitor glucose levels in individuals with diabetes.

A typical glucose sensor works according to the following chemical reactions:

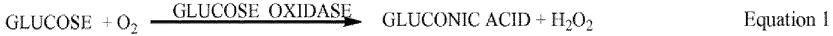

H₂O₂ → O₂ + 2H⁺ + 2 e⁻ Equation 2

The glucose oxidase is used to catalyze the reaction between glucose and oxygen to yield gluconic acid and hydrogen peroxide as shown in equation 1. The H₂O₂ reacts electrochemically as shown in equation 2, and the current is measured by a potentiostat. The stoichiometry of the reaction provides challenges to developing *in vivo* sensors. In particular, for optimal sensor performance, sensor signal output should be determined only by the analyte of interest (glucose), and not by any co-substrates (O₂) or kinetically controlled parameters such as diffusion. If oxygen and glucose are present in equimolar concentrations, then the H₂O₂ is stoichiometrically related to the amount of glucose that reacts at the enzyme; and the associated current that generates the sensor signal is proportional to the amount of glucose that reacts with the enzyme. If, however, there is insufficient oxygen for all of the glucose to react with the enzyme, then the current will be proportional to the oxygen concentration, not the glucose concentration. Consequently, for the sensor to provide a signal that depends solely on the concentrations of glucose, glucose must be the limiting reagent, i.e. the O₂ concentration must be in excess for all potential glucose concentrations. A problem with using such glucose sensors *in vivo,* however, is that the oxygen concentration where the sensor is implanted *in vivo is* low relative to glucose, a phenomena which can compromise the accuracy of sensor readings.

There are a number of approaches to solving the oxygen deficit problem. One is to make a porous membrane from a fully oxygen permeable material. However, the small amount of enzyme disposed for glucose tends to become inactivated (see, e.g. U. S. patent No 4,484,987). Another approach is to use a homogenous polymer membrane with hydrophobic and hydrophilic regions that control oxygen and glucose permeability (see, e.g. U. S. patent Nos. 5,428,123; 5,322,063, 5,476,094). For example, Van Antwerp et al. have developed linear polyurea membranes comprising silicone hydrophobic components that allow for a high oxygen permeability in combination with hydrophilic component that allow for a limited glucose permeability (see e.g. U.S. Patent Nos. 5,777,060, 5,882,494 and 6,642,015). Methods and materials that facilitate the use of such membranes are desirable.

### SUMMARY OF THE INVENTION

Amperometric sensors such as glucose sensors that are used by diabetics often incorporate polymeric membranes in order to control the diffusion of glucose and/or other compounds. Unfortunately, the molecular weight and permeability of these polymeric membranes can be reduced over time due to phenomena such as heat, oxidation and e-beam sterilization. Such phenomenon negatively impact a number of sensor characteristics including sensor stability and performance. Embodiments of the invention include biocompatible membranes that exhibit material properties that allow them to be used in a biosensor in order to control analyte (e.g. glucose) permeability. These compositions further exhibit a combination of new desirable properties including an increased resistance to thermal and oxidative degradation. As discussed in detail below, glucose sensors that incorporate such robust polymeric membranes show an extended shelf life as well as an enhanced performance profile.
The invention disclosed herein has a number of embodiments. One embodiment of the invention is the use of a biocompatible membrane composition comprising a polymer formed from a mixture comprising: a diisocyanate; a siloxane; a hydrophilic diol or hydrophilic diamine; and a polymer stabilizing compound. Typically, the polymer stabilizing compound has a molecular weight of less than 1000 g/mol; and further comprises a benzyl ring having a hydroxyl moiety (ArOH). In certain embodiments, the polymer stabilizing compound comprises at least two benzyl rings having at least one hydroxyl moiety. In typical embodiments of the invention, the stabilizing agent comprises a free radical scavenger. Embodiments of the invention include those which use antioxidant compounds as well as strong reducing agents that can adsorb and bind oxygen. Illustrative polymer stabilizing compounds include for example pyrogallol, catechol, 2,2'-Methylenebis(6-tert-butyl-4-methylphenol, 2,2'-Ethylene-bis (4,6-di-tert-butylphenol), and 4,4'-Methylenebis(2,6-di-tert-butylphenol). Such polymer compositions can be formed, for example, from a mixture comprising: 45 - 55 mol% diisocyanate; 10-30 mol% siloxane; 30-45 mol% hydrophilic diol or hydrophilic diamine; and 0.1-5 weight % of the polymer stabilizing compound.

As disclosed in detail below, the use of the compositions of the invention can be manipulated to include further characteristics that are useful in a variety of contexts, for example, as biocompatible glucose limiting membranes. In some embodiments of the invention, the polymer stabilizing compound is covalently coupled to a terminal end of the polymer. In other embodiments, the polymer stabilizing compound is not covalently coupled to the polyurethane/polyurea polymer, and is instead physically entrapped within a plurality of polyurethane/polyurea polymers. In certain embodiments of the invention the composition is combined with one or more further compounds, for example, one comprising a platinum or iridium composition (e.g. where this polymer is disposed, along with other layers of material, on a platinum or iridium electrode). In other embodiments of the invention, the stabilized polymer compositions are further mixed with another polymer, for example a branched acrylate polymer.

Another embodiment of the invention is a device that incorporates a stabilized polymer composition as disclosed herein, for example an amperometric analyte sensor system (e.g. a glucose sensor used by diabetic individual). Such systems typically include, for example, a working electrode, a counter electrode; and a reference electrode; and an analyte sensing layer disposed on the working electrode. In addition such systems further include an analyte modulating layer disposed on the analyte sensing layer, wherein the analyte modulating layer comprises a polyurethane/polyurea polymer formed from a mixture comprising: a diisocyanate; a hydrophilic polymer comprising a hydrophilic diol or hydrophilic diamine; a siloxane having an amino, hydroxyl or carboxylic acid functional group at a terminus; and a polyurethane/polyurea polymer stabilizing compound selected for its ability to inhibit thermal and oxidative degradation of polyurethane/polyurea polymers formed from the mixture, wherein the polyurethane/polyurea polymer stabilizing compound has a molecular weight of less than 1000 g/mol; and comprises a benzyl ring having a hydroxyl moiety (ArOH). In typical embodiments of the invention, the polyurethane/polyurea polymer stabilizing compound exhibits an antioxidant activity (e.g. embodiments that comprise phenolic antioxidants). Optionally, the polyurethane/polyurea polymer stabilizing compound comprises at least two benzyl rings having a hydroxyl moiety.

In certain embodiments of the invention, a stabilizing compound is selected so that the analyte modulating layer comprising the polyurethane/polyurea polymer stabilizing compound exhibits an increased permeability to glucose as compared to analyte modulating layer not comprising the polyurethane/polyurea polymer stabilizing compound. Optionally in such systems, the polyurethane/polyurea polymer stabilizing compound is covalently coupled to the polyurethane/polyurea polymer. Alternatively, the polyurethane/polyurea polymer stabilizing compound is not covalently coupled to the polyurethane/polyurea polymer; and is instead entrapped within a plurality of polyurethane/polyurea polymers. In an exemplary embodiment of the invention, the polyurethane/polyurea polymer comprises 45-55 mol% diisocyanate (e.g. a hexamethylene diisocyanate); 10-30 mol% siloxane (e.g. a polymethylhydrosiloxane); 30-45 mol% hydrophilic diol or hydrophilic diamine (e.g. Jeffamine 600); and 0.1-5 weight % polyurethane/polyurea polymer stabilizing compound (e.g. pyrogallol, catechol, 2,2'-Methylenebis(6-tert-butyl-4-methylphenol, 2,2'-Ethylene-bis(4,6-di-tert-butylphenol), or 4,4'-Methylenebis(2,6-di-tert-butylphenol).

The analyte sensor systems of the invention that comprise the improved membrane compositions can further include additional elements that facilitate the function of such compositions, for example an interference rejection membrane; a protein layer; an adhesion promoting layer disposed on the analyte sensing layer, wherein the adhesion promoting layer promotes the adhesion between the analyte sensing layer and the analyte modulating layer; or a cover layer wherein the cover layer comprises an aperture positioned on the cover layer so as to facilitate an analyte present in the mammal contacting and diffusing through an analyte modulating layer; and contacting the analyte sensing layer. In certain embodiments of the invention the analyte sensor system includes a probe platform and the first probe comprises a first electrode array comprising a working electrode, a counter electrode and a reference electrode; and a second electrode array comprising a working electrode, a counter electrode and a reference electrode. Some embodiments of the invention can include a second probe coupled to the probe platform and adapted to be inserted in vivo, wherein the second probe comprises a third electrode array comprising a working electrode, a counter electrode and a reference electrode; and a fourth electrode array comprising a working electrode, a counter electrode and a reference electrode. Typically in such embodiments, the first, second, third and fourth electrode arrays are configured to be electronically independent of one another. Optionally at least two of the working electrodes in the different electrode arrays are coated with analyte modulating layers formed from different reaction mixtures having different material properties.

As discussed in detail below, other embodiments of the invention include methods for making sensors comprising membranes formed from the stabilized polymer compositions disclosed herein as well as methods of using such sensors to sense analytes such as glucose. Other objects, features and advantages of the present invention will become apparent to those skilled in the art from the following detailed description. It is to be understood, however, that the detailed description and specific examples, while indicating some embodiments of the present invention are given by way of illustration and not limitation. Many changes and modifications within the scope of the present invention may be made without departing from the spirit thereof, and the invention includes all such modifications.

### BRIEF DESCRIPTION OF THE FIGURES

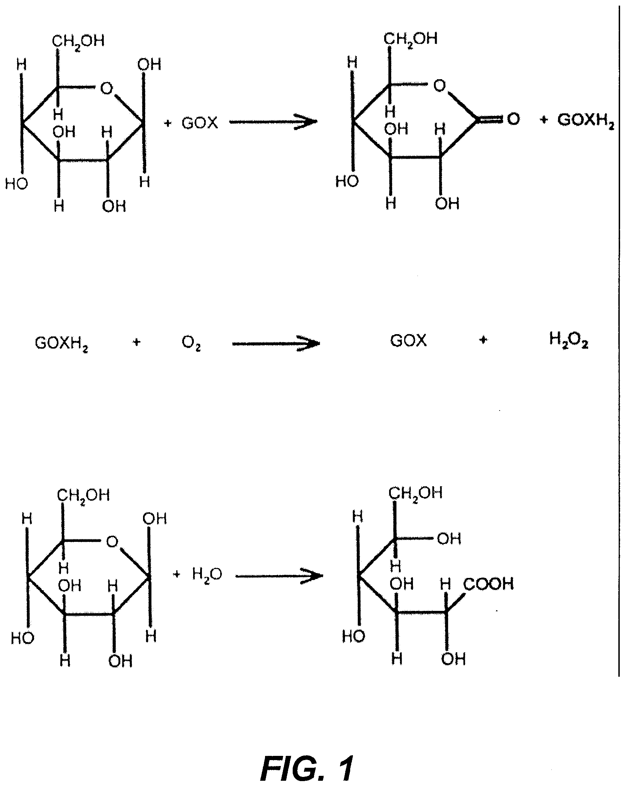

**FIG. 1** provides a schematic of the well known reaction between glucose and glucose oxidase. As shown in a stepwise manner, this reaction involves glucose oxidase (GOx), glucose and oxygen in water. In the reductive half of the reaction, two protons and electrons are transferred from β-D-glucose to the enzyme yielding d-gluconolactone. In the oxidative half of the reaction, the enzyme is oxidized by molecular oxygen yielding hydrogen peroxide. The d-gluconolactone then reacts with water to hydrolyze the lactone ring and produce gluconic acid. In certain electrochemical sensors of the invention, the hydrogen peroxide produced by this reaction is oxidized at the working electrode (H₂O₂ → 2H+ + O₂ + 2e⁻).

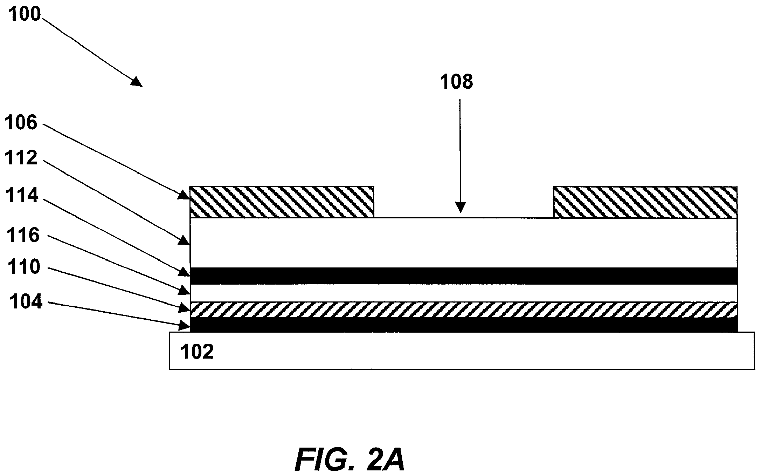

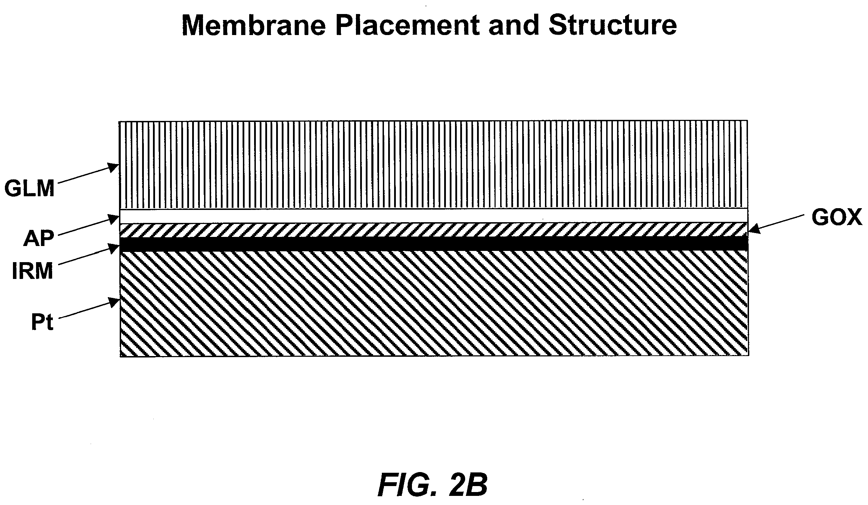

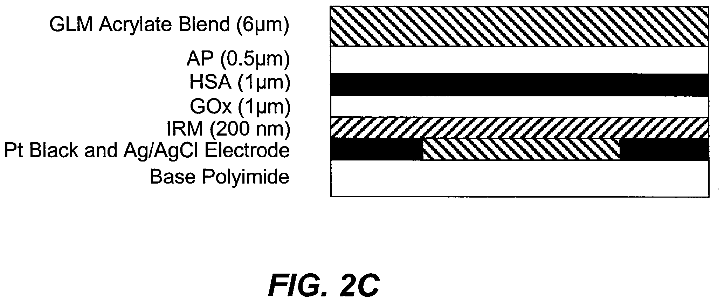

**FIG. 2A** provides a diagrammatic view of one embodiment of an amperometric analyte sensor comprising a membrane formed from a polyurethane/polyurea analyte modulating composition (112). Typically this composition is a glucose limiting polymer (GLP) that functions as a glucose limiting membrane (GLM). **FIG. 2B** provides a diagrammatic view of one embodiment of an amperometric glucose sensor comprising both glucose oxides (GOx) and a glucose limiting membrane. **FIG. 2C** provides a diagrammatic view of a specific embodiment of an amperometric glucose sensor having a plurality of layers including a layer of a glucose limiting membrane (GLM, made from glucose limiting polymer compositions), a layer of an adhesion promoter, a layer of human serum albumin (HSA), a layer of glucose oxidase, a layer of an interference rejection membrane (IRM), and an electrode layer, all of which are supported by a base comprised of a polyimide composition.

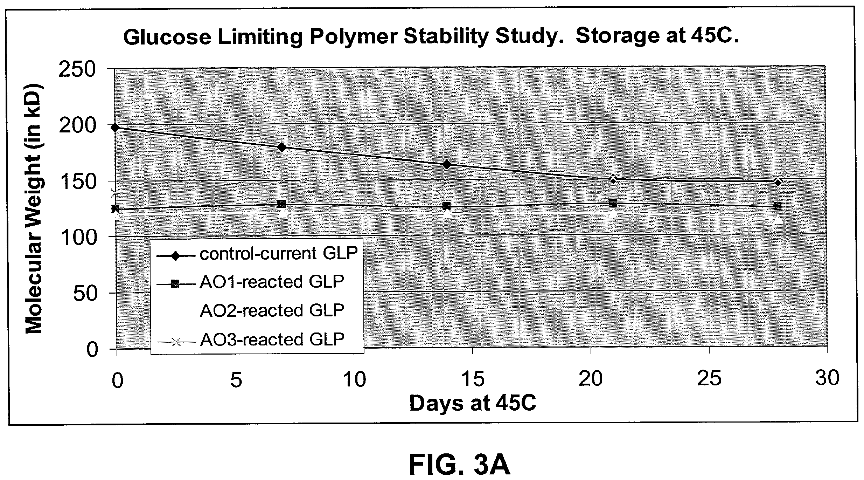

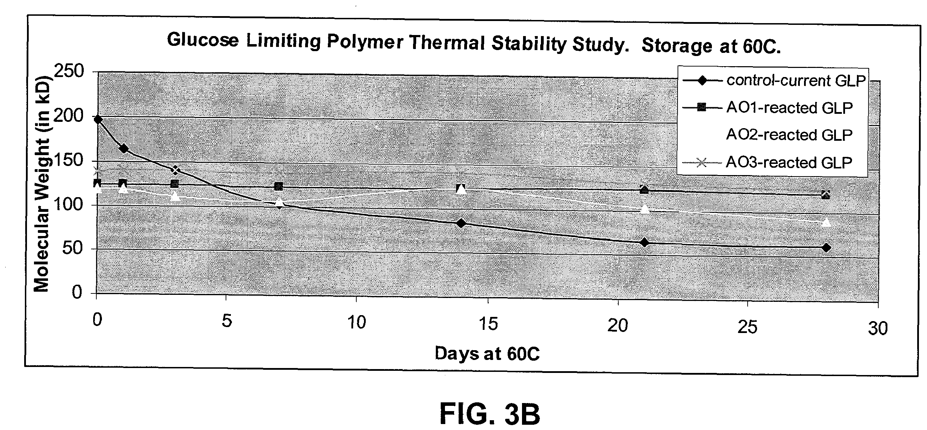

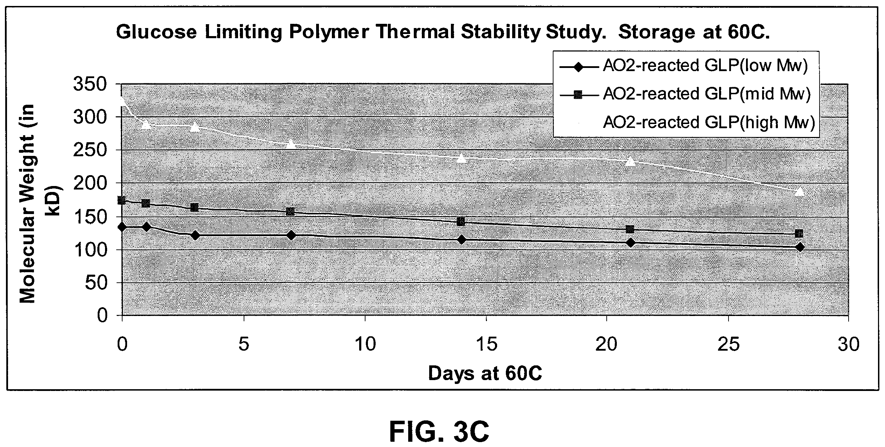

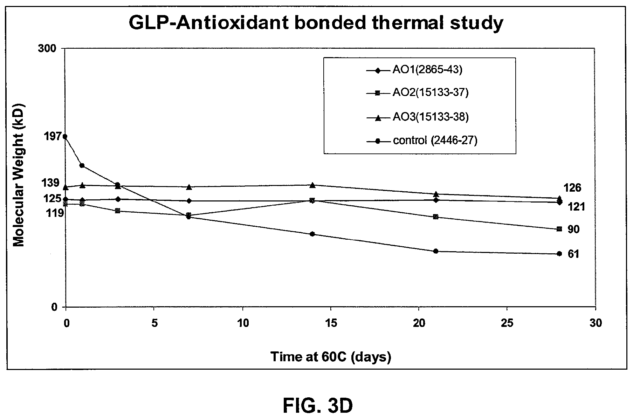

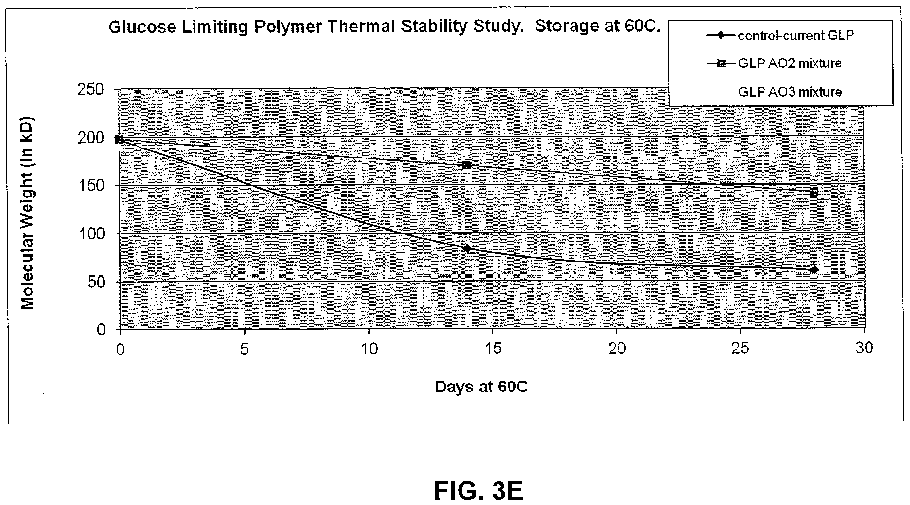

**FIGS 3A-3E** provide graphs showing the thermal stability of polyurethane/polyurea glucose limiting polymer (GLP) compositions to which various stabilizing compounds have been added. The polymer compositions shown in these figures are further described in Example 1 below. **FIG. 3A** provides a graph showing that GLP compositions that do not comprise stabilizing compounds ("control-current") drop in molecular weight by 25% (197kD to 148kD) after 4 weeks of storage at 45°C. In comparison, GLP's combined with stabilizing compounds show much less molecular weight decrease (<5%) over the same period of time (in these reacted embodiments, the stabilizing compounds are bound to the polymers via covalent bonds). Illustrating this, AO1-reacted GLP (stabilized with 4,4'-Methylenebis(2,6-di-tert-butylphenol), CAS# 118-82-1) drops only 1% (125kD to 124kD), AO2-reacted GLP drops 4% (119kD to 114kD), and AO3-reacted GLP shows no detectable change in molecular weight (139kD to 150kD). **FIG. 3B** provides a graph showing show the GLP molecular weight drops dramatically (69%) after one month of storage at 60°C, while in comparison, 3 antioxidant stabilizing compound reacted GLP's show much improved thermal stabilities. In particular, AO1-reacted GLP drops only 3% (125kD to 121kD), AO2-reacted GLP drops 24% (199kD to 90kD), and AO3 drops 9% (139kD to 126kD). **FIG. 3C** provides a graph showing the thermal stability of 3 AO2-reacted polymers of different molecular weight: low Mw (135kD), medium Mw (172kD), and high Mw (324kD) polymers at 60°C and provides evidence that the antioxidant stabilizing compound is in fact responsible for most of the stabilizing effect, rather than the lower initial molecular weight. The very high molecular weight antioxidant-reacted GLP (324kD) drops only 42% after one month, versus 69% for the current GLP (initial Mw = 197kD). The low Mw (135kD) AO2-reacted GLP shows only a 24% decrease (135kD to 103kD) that is similar to the decrease (28%) of the medium Mw (172kD to 124kD) GLP. Again, both show a significant improvement over a GLP formulation that lacks such stabilizing compounds (which decreases in MW BY 69%). **FIG. 3D** provides a graph showing the thermal stability of GLP polymers covalently bound to stabilizing compounds and a control GLP that lacks such stabilizing compounds. **FIG. 3E** provides a graph showing the thermal stability of GLP polymers mixed with stabilizing compounds and a control GLP that lacks such stabilizing compounds. In these experiments, the stabilizing agent is not covalently bonded to the polymers but is instead mixed and entrapped within the polymeric matrix.

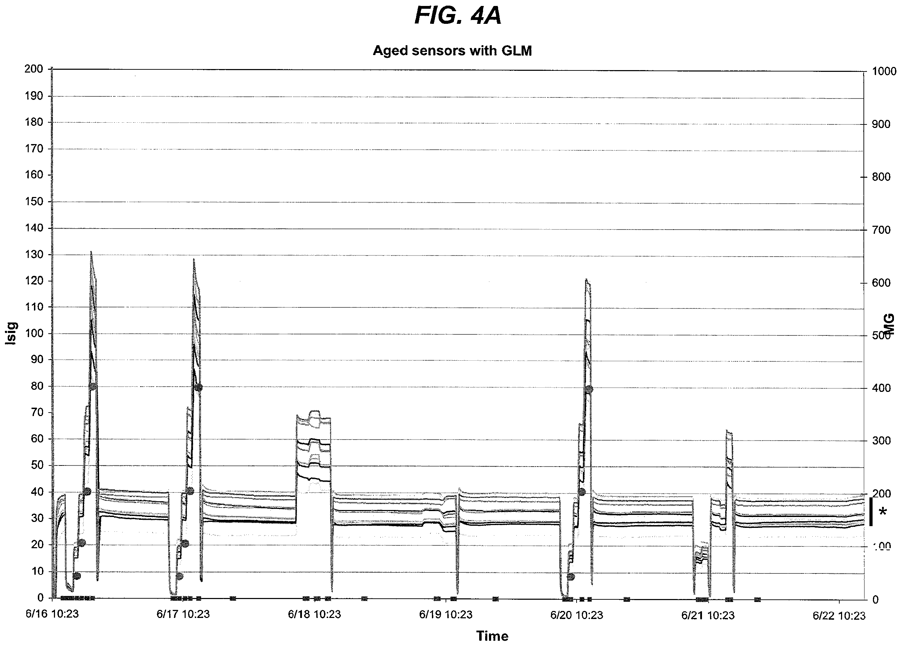

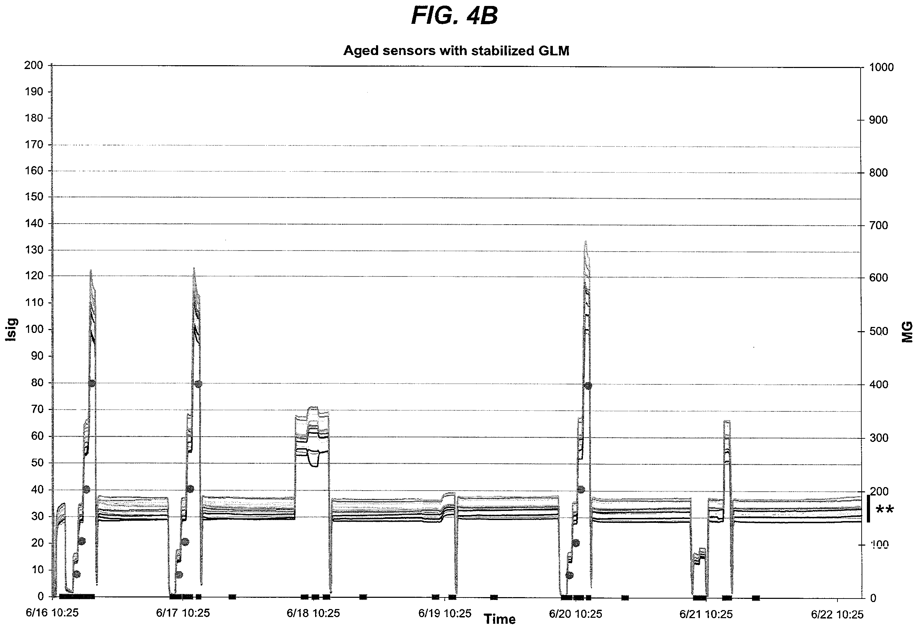

**FIGS 4A****-and 4B** provide graphs showing the results of an accelerated aging study in which groups of sensors were heated at 45 degrees centigrade for 4.7 months. **FIG. 4A** shows studies from a group of glucose sensors formed using a conventional GLM composition to which no polyurethane/polyurea polymer stabilizing compound was added. **FIG. 4B** shows studies from a group of sensors formed using a GLM composition to which a polyurethane/polyurea polymer stabilizing compound has been added (in this embodiment, the compound is covalently bound to the polymers in this composition). Effects of the stabilizing compound can be observed, for example, by comparing the range of individual sensor Isig values in the sensors shown in FIG. 4A ("*") as compared the range of individual sensor Isig values in the sensors shown in FIG. 4B ("**").

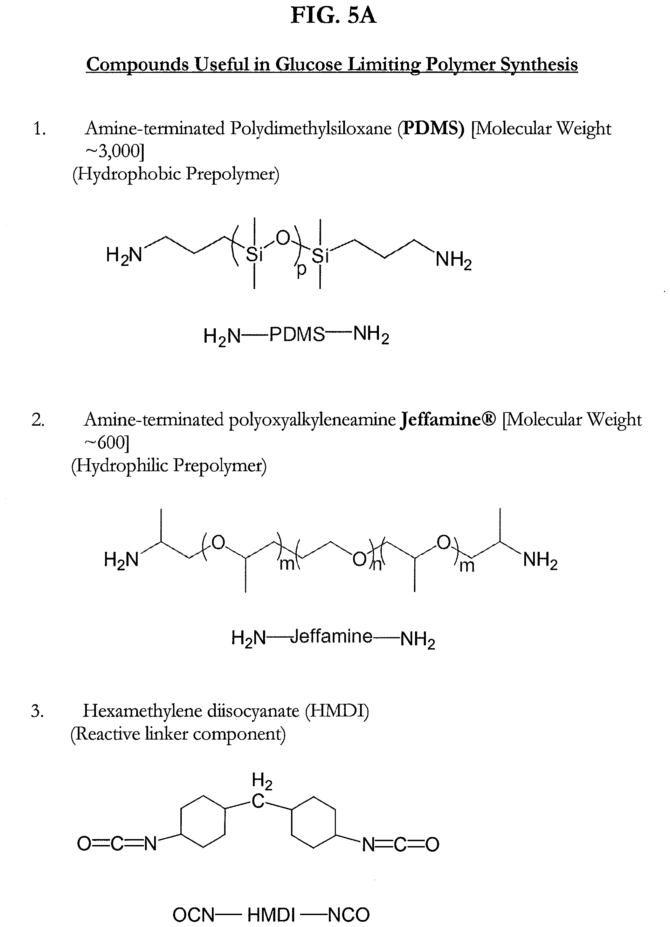

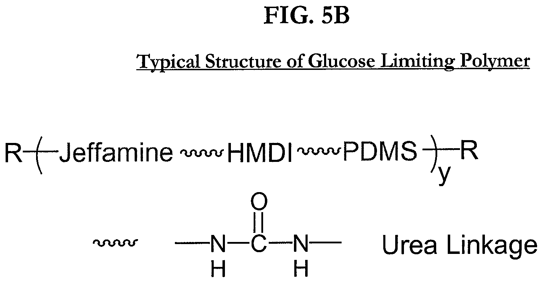

**FIG. 5A** shows compounds useful in the synthesis of polymers useful in embodiments of the invention, for example as a glucose limiting membrane in amperometric glucose sensors. **FIG. 5B** shows an illustrative polymer structure formed from the compounds shown in FIG. 5A.

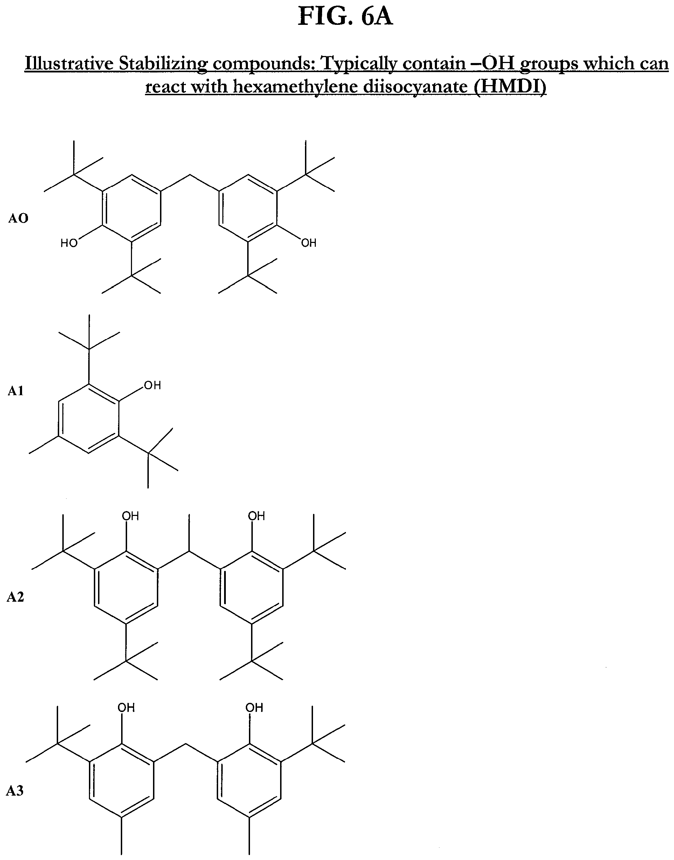

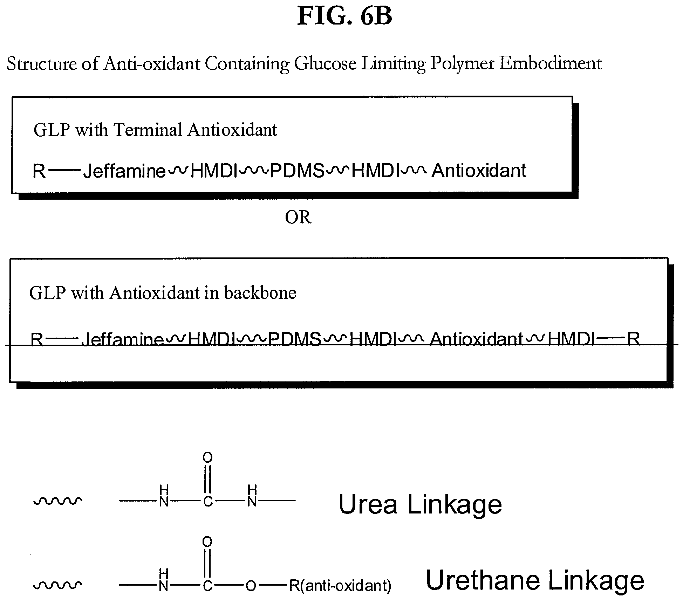

**FIG. 6A** shows illustrative stabilizing compounds useful in the synthesis of polymers of the invention. These compounds typically comprise -OH moieties which can, for example, react with hexamethylene diisocyanate. **FIG. 6B** shows an illustrative polymer structures formed from the compounds shown in FIG. 6A.

### DETAILED DESCRIPTION OF THE EMBODIMENTS

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted. A number of terms are defined below. (see, e.g. Wright et al., J. Am. Chem. Soc., 123, 1173-1183 (2001); Han et al., Polymer 38(2): 317-323 (1997); Chimi et al., JAOCS 68(5): 307-312 (1991); and Yeh et al., Colloids and Surfaces B: Biointerfaces 59:67-73(2007)). Publications cited herein are cited for their disclosure prior to the filing date of the present application.

It must be noted that as used herein and in the appended claims, the singular forms "a", "and", and "the" include plural referents unless the context clearly dictates otherwise. Thus, for example, reference to "a stabilizing compound"" includes a plurality of such compounds and equivalents thereof known to those skilled in the art, and so forth. All numbers recited in the specification and associated claims that refer to values that can be numerically characterized with a value other than a whole number (e.g. "50 mol%") are understood to be modified by the term "about".

The term "analyte" as used herein is a broad term and is used in its ordinary sense, including, without limitation, to refer to a substance or chemical constituent in a fluid such as a biological fluid (for example, blood, interstitial fluid, cerebral spinal fluid, lymph fluid or urine) that can be analyzed. Analytes can include naturally occurring substances, artificial substances, metabolites, and/or reaction products. In some embodiments, the analyte for measurement by the sensing regions, devices, and methods is glucose. However, other analytes are contemplated as well, including but not limited to, lactate. Salts, sugars, proteins fats, vitamins and hormones naturally occurring in blood or interstitial fluids can constitute analytes in certain embodiments. The analyte can be naturally present in the biological fluid or endogenous; for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin. The metabolic products of drugs and pharmaceutical compositions are also contemplated analytes.

The term "sensor," as used herein, is a broad term and is used in its ordinary sense, including, without limitation, the portion or portions of an analyte-monitoring device that detects an analyte. In one embodiment, the sensor includes an electrochemical cell that has a working electrode, a reference electrode, and optionally a counter electrode passing through and secured within the sensor body forming an electrochemically reactive surface at one location on the body, an electronic connection at another location on the body, and a membrane system affixed to the body and covering the electrochemically reactive surface. During general operation of the sensor, a biological sample (for example, blood or interstitial fluid), or a portion thereof, contacts (directly or after passage through one or more membranes or domains) an enzyme (for example, glucose oxidase); the reaction of the biological sample (or portion thereof) results in the formation of reaction products that allow a determination of the analyte level in the biological sample.

Embodiments of the invention disclosed herein provide sensors of the type used, for example, in subcutaneous or transcutaneous monitoring of blood glucose levels in a diabetic patient. A variety of implantable, electrochemical biosensors have been developed for the treatment of diabetes and other life-threatening diseases. Many existing sensor designs use some form of immobilized enzyme to achieve their bio-specificity. Embodiments of the invention described herein can be adapted and implemented with a wide variety of known electrochemical sensors, including for example, U.S. Patent Application No. 20050115832, U.S. Pat. Nos. 6,001,067, 6,702,857, 6,212,416, 6,119,028, 6,400,974, 6,595,919, 6,141,573, 6,122,536, 6,512,939 5,605,152, 4,431,004, 4,703,756, 6,514,718, 5,985,129, 5,390,691, 5,391, 250, 5,482,473, 5,299,571, 5,568,806, 5,494,562, 6,120,676, 6,542,765 as well as PCT International Publication Numbers WO 01/58348, WO 04/021877, WO 03/034902, WO 03/035117, WO 03/035891, WO 03/023388, WO 03/022128, WO 03/022352, WO 03/023708, WO 03/036255, WO03/036310 WO 08/042625, and WO 03/074107, and European Patent Application EP 1153571.

As discussed in detail below, embodiments of the invention disclosed herein provide sensor elements having enhanced material properties and/or architectural configurations and sensor systems (e.g. those comprising a sensor and associated electronic components such as a monitor, a processor and the like) constructed to include such elements. The disclosure further provides methods for making and using such sensors and/or architectural configurations. While some embodiments of the invention pertain to glucose sensors, a variety of the elements disclosed herein (e.g. analyte modulating membranes made from stabilized polymeric compositions) can be adapted for use with any one of the wide variety of sensors known in the art. The analyte sensor elements, architectures and methods for making and using these elements that are disclosed herein can be used to establish a variety of layered sensor structures. Such sensors of the invention exhibit a surprising degree of flexibility and versatility, characteristics which allow a wide variety of sensor configurations to be designed to examine a wide variety of analyte species.

Specific aspects of embodiments of the invention are discussed in detail in the following sections.

### I. TYPICAL ELEMENTS, CONFIGURATIONS AND ANALYTE SENSORS OF THE INVENTION

### A. OPTIMIZED SENSOR ELEMENTS OF THE INVENTION

A wide variety of sensors and sensor elements are known in the art including amperometric sensors used to detect and/or measure biological analytes such as glucose. Many glucose sensors are based on an oxygen (Clark-type) amperometric transducer (see, e.g. Yang et al., Electroanalysis 1997, 9, No. 16: 1252-1256; Clark et al., Ann. N.Y. Acad. Sci. 1962, 102, 29; Updike et al., Nature 1967, 214,986; and Wilkins et al., Med. Engin. Physics, 1996, 18, 273.3-51). Electrochemical glucose sensors that utilize the chemical reaction between glucose and glucose oxidase to generate a measurable signal typically include polymeric compositions that modulate the diffusion of analytes including glucose in order to overcome what is known in the art as the "oxygen deficit problem". Specifically, because glucose oxidase based sensors require both oxygen (O₂) as well as glucose to generate a signal, the presence of an excess of oxygen relative to glucose, is necessary for the operation of a glucose oxidase based glucose sensor. However, because the concentration of oxygen in subcutaneous tissue is much less than that of glucose, oxygen can be the limiting reactant in the reaction between glucose, oxygen, and glucose oxidase in a sensor, a situation which compromises the sensor's ability to produce a signal that is strictly dependent on the concentration of glucose. Material modifications to polymeric compositions having a specified function can be problematical in that such modifications can result in unpredictable alterations in the crucial permselective properties of these membranes. For example, because the properties of a material can influence the rate at which compounds diffuse through that material to the site of a measurable chemical reaction, the material properties of an analyte modulating layer used in electrochemical glucose sensors that utilize the chemical reaction between glucose and glucose oxidase to generate a measurable signal, should not for example, favor the diffusion of glucose over oxygen in a manner that contributes to the oxygen deficit problem. In this context, the use of the stabilized polymeric compositions disclosed herein maintain an ability to address the oxygen deficit problem observed in glucose sensors while simultaneously providing such sensors with further advantageous properties including an extended shelf life as well as an enhanced performance profile.

As discussed in detail below, embodiments of the invention relate to the use of an electrochemical sensor that exhibits a novel constellation of elements including a stabilized polymeric membrane having a unique set of technically desirable material properties. The electrochemical sensors of the invention are designed to measure a concentration of an analyte of interest (e.g. glucose) or a substance indicative of the concentration or presence of the analyte in fluid. In some embodiments, the sensor is a continuous device, for example a subcutaneous, transdermal, or intravascular device. In some embodiments, the device can analyte a plurality of intermittent blood samples. The sensor embodiments disclosed herein can use any known method, including invasive, minimally invasive, and non-invasive sensing techniques, to provide an output signal indicative of the concentration of the analyte of interest. Typically, the sensor is of the type that senses a product or reactant of an enzymatic reaction between an analyte and an enzyme in the presence of oxygen as a measure of the analyte in vivo or in vitro. Such sensors comprise a stabilized polymeric membrane surrounding the enzyme through which an analyte migrates prior to reacting with the enzyme. The product is then measured using electrochemical methods and thus the output of an electrode system functions as a measure of the analyte.

Embodiments of the invention include for example a sensor having a plurality of layered elements including an analyte limiting membrane comprising a stabilized polymeric composition. Such polymeric membranes are particularly useful in the construction of electrochemical sensors for *in vivo* use. The membrane embodiments of the invention allow for a combination of desirable properties including: an enhanced lifetime profile as well as a permeability profile to molecules such as glucose that allow them to, for example address the oxygen deficit problem. In addition, these polymeric membranes exhibit good mechanical properties for use as an outer polymeric membrane. Consequently, glucose sensors that incorporate such polymeric membranes show a highly desirable *in-vivo* performance profile.

The invention disclosed herein has a number of embodiments. One embodiment of the invention is the use of a biocompatible membrane composition comprising a polymer formed from a mixture comprising: a diisocyanate; a siloxane; a hydrophilic diol or hydrophilic diamine; and a polymer stabilizing compound. Typically, the polymer stabilizing compound has a molecular weight of less than 1000 g/mol; and further comprises a benzyl ring having at least one hydroxyl moiety (ArOH). In some embodiments, the polymer stabilizing compound comprises at least two (or three or four) benzyl rings having at least one hydroxyl moiety. In certain embodiments, a benzene ring of the stabilizing compound comprises two or three or more hydroxyl moieties. Embodiments of the invention include those which use antioxidant compounds as well as strong reducing agents that can adsorb and bind oxygen. Some illustrative polymer stabilizing compounds include for example 4,4'-Methylenebis(2,6-di-tert-butylphenol), 2,2'-Ethylidene-bis(4,6-di-tert-butylphenol), 2,2'-Methylenebis (6-tert-butyl-4-methylphenol), Pyrogallol (also 1,2,3-Trihydroxybenzene), 2,6-Di-tert-butyl-4-methylphenol, 2,4,6-Tri-tert-butylphenol and 4,4'-Isopropylidenedicyclohexanol. Using the polymer synthesis methods and accelerated aging protocols as disclosed for example in the Examples below, those of skill in this art can assess the stabilizing properties of a compound with only a minimal amount experimentation. Contemplated further stabilizing compounds include a oxygen absorbent composition such as ascorbic acid, isoascorbic acid, gallic acid, tocopherol, hydroquinone, catechol, resorcine, dibutylhydroxyltoluene, dibutylhydroxyanisole (see also the compounds disclosed in U.S. Patent No. 5,143,763). In certain embodiments of the invention, a stabilizing compound is selected for its ability to increase permeability to glucose as compared to analyte modulating layer not comprising the polyurethane/polyurea polymer stabilizing compound. Unexpectedly, stabilizing molecules that are hydrophobic can increase a composition's permeability to glucose, a hydrophilic molecule. Without being bound by a specific theory or mechanism of action, this surprising increase in glucose permeability in polymers stabilized with such molecules (compositions which nonetheless can be used to form membranes designed to address the oxygen deficit problem in glucose sensors) may results from such molecules steric effects on the polymer network.

As disclosed in detail below, the use of the compositions of the invention can be manipulated to include further characteristics that are useful in a variety of contexts, for example, as biocompatible glucose limiting membranes. In some embodiments of the invention, the polymer stabilizing compound is covalently coupled to a terminal end of the polymer. Such embodiments can, for example address issues where the specific characteristics of a particular polymer or stabilizing agent as such that covalent bonds at sites other than the terminal ends can have undesirable effects. In other embodiments, the polymer stabilizing compound is not covalently coupled to the polyurethane/polyurea polymer, and is instead physically entrapped within a plurality of polyurethane/polyurea polymers. In certain embodiments of the use of the composition is combined with one or more further compounds, for example, one comprising a platinum or iridium composition (e.g. where this polymer is disposed, along with other layers of material, on a platinum or iridium electrode). In other embodiments of the invention, the stabilized polymer compositions are further mixed with another polymer, for example a branched acrylate polymer.

Another embodiment of the invention is a device that incorporates a stabilized polymer composition as disclosed herein, for example an amperometric analyte sensor system (e.g. a glucose sensor system used by diabetic individual). Such systems typically include, for example, a working electrode, a counter electrode; and a reference electrode; and an analyte sensing layer disposed on the working electrode. In addition such systems further include an analyte modulating layer disposed on the analyte sensing layer, wherein the analyte modulating layer comprises a polyurethane/polyurea polymer formed from a mixture comprising: a diisocyanate; a hydrophilic polymer comprising a hydrophilic diol or hydrophilic diamine; a siloxane having an amino, hydroxyl or carboxylic acid functional group at a terminus; and a polyurethane/polyurea polymer stabilizing compound selected for its ability to inhibit thermal and oxidative degradation of polyurethane/polyurea polymers formed from the mixture, wherein the polyurethane/polyurea polymer stabilizing compound has a molecular weight of less than 1000 g/mol; and comprises a benzyl ring having a hydroxyl moiety (ArOH). In typical embodiments of the invention, the polyurethane/polyurea polymer stabilizing compound exhibits an antioxidant activity (e.g. embodiments that comprise phenolic antioxidants). Optionally, the polyurethane/polyurea polymer stabilizing compound comprises at least two benzyl rings having a hydroxyl moiety.

From the above description, it will be apparent to one of skill in the art that the discovery underlying the present invention is the use of stabilized polymeric compositions, in the formation of biocompatible membranes. Membrane embodiments produced from these components are homogeneous and are useful for coating a number of biosensors and devices designed for subcutaneous implantation. Descriptions of stabilized linear polyurea/polyurethane polymer compositions and other elements useful in the design of biosensors are provided below.

### LINEAR POLYURETHANE/POLYUREA POLYMERS

One polymeric composition used in embodiments of the present invention is a polyurethane/polyurea polymer. As used herein, the term "polyurethane/polyurea polymer" refers to a polymer containing urethane linkages, urea linkages or combinations thereof. As is known in the art, polyurethane is a polymer consisting of a chain of organic units joined by urethane (carbamate) links. Polyurethane polymers are typically formed through step-growth polymerization by reacting a monomer containing at least two isocyanate functional groups with another monomer containing at least two hydroxyl (alcohol) groups in the presence of a catalyst. Polyurea polymers are derived from the reaction product of an isocyanate component and a diamine. Typically, such polymers are formed by combining diisocyanates with alcohols and/or amines. For example, combining isophorone diisocyanate with PEG 600 and aminopropyl polysiloxane under polymerizing conditions provides a polyurethane/polyurea composition having both urethane (carbamate) linkages and urea linkages. Such polymers are well known in the art and described for example in U.S. Patent Nos. 5,777,060, 5,882,494 and 6,632,015, and PCT publications WO 96/30431; WO 96/18115; WO 98/13685; and WO 98/17995.

The polyurethane/polyurea compositions of the invention are prepared from biologically acceptable polymers whose hydrophobic/hydrophilic balance can be varied over a wide range to control the ratio of the diffusion coefficient of oxygen to that of glucose, and to match this ratio to the design requirements of electrochemical glucose sensors intended for in vivo use. Such compositions can be prepared by conventional methods by the polymerization of monomers and polymers noted above. The resulting polymers are soluble in solvents such as acetone or ethanol and may be formed as a membrane from solution by dip, spray or spin coating.

Diisocyanates useful in this embodiment of the invention are those which are typically those which are used in the preparation of biocompatible polyurethanes. Such diisocyanates are described in detail in Szycher, SEMINAR ON ADVANCES IN MEDICAL GRADE POLYURETHANES, Technomic Publishing, (1995) and include both aromatic and aliphatic diisocyanates. Examples of suitable aromatic diisocyanates include toluene diisocyanate, 4,4'-diphenylmethane diisocyanate, 3,3'-dimethyl-4,4'-biphenyl diisocyanate, naphthalene diisocyanate and paraphenylene diisocyanate. Suitable aliphatic diisocyanates include, for example, 1,6hexamethylene diisocyanate (HDI), trimethylhexamethylene diisocyanate (TMDI), trans1,4-cyclohexane diisocyanate (CHDI), 1,4-cyclohexane bis(methylene isocyanate) (BDI), 1,3-cyclohexane bis(methylene isocyanate) (H6 XDI), isophorone diisocyanate (IPDI) and 4,4'-methylenebis(cyclohexyl isocyanate) (H2 MDI). In some embodiments, the diisocyanate is isophorone diisocyanate, 1,6-hexamethylene diisocyanate, or 4,4'methylenebis(cyclohexyl isocyanate). A number of these diisocyanates are available from commercial sources such as Aldrich Chemical Company (Milwaukee, Wis., USA) or can be readily prepared by standard synthetic methods using literature procedures.
The quantity of diisocyanate used in the reaction mixture for the polyurethane/polyurea polymer compositions is typically 50 mol % relative to the combination of the remaining reactants. More particularly, the quantity of diisocyanate employed in the preparation of the polyurethane/polyurea polymer will be sufficient to provide at least about 100% of the --NCO groups necessary to react with the hydroxyl or amino groups of the remaining reactants. For example, a polymer which is prepared using x moles of diisocyanate, will use a moles of a hydrophilic polymer (diol, diamine or combination), b moles of a silicone polymer having functionalized termini, and c moles of a chain extender, such that x=a+b+c, with the understanding that c can be zero.

Another reactant used in the preparation of the polyurethane/polyurea polymers described herein is a hydrophilic polymer. The hydrophilic polymer can be a hydrophilic diol, a hydrophilic diamine or a combination thereof. The hydrophilic diol can be a poly(alkylene)glycol, a polyester-based polyol, or a polycarbonate polyol. As used herein, the term "poly(alkylene)glycol" refers to polymers of lower alkylene glycols such as poly(ethylene)glycol, poly(propylene)glycol and polytetramethylene ether glycol (PTMEG). The term "polyester-based polyol" refers to a polymer in which the R group is a lower alkylene group such as ethylene, 1,3-propylene, 1,2-propylene, 1,4-butylene,2,2-dimethyl-1,3-propylene, (e.g. as depicted in FIG. 4 of U.S. Patent Nos. 5,777,060). One of skill in the art will also understand that the diester portion of the polymer can also vary from the six-carbon diacid shown. For example, while FIG. 4 of U.S. Patent Nos. 5,777,060 illustrates an adipic acid component, the present invention also contemplates the use of succinic acid esters, glutaric acid esters. The term "polycarbonate polyol" refers those polymers having hydroxyl functionality at the chain termini and ether and carbonate functionality within the polymer chain. The alkyl portion of the polymer will typically be composed of C2 to C4 aliphatic radicals, or in some embodiments, longer chain aliphatic radicals, cycloaliphatic radicals or aromatic radicals. The term "hydrophilic diamines" refers to any of the above hydrophilic diols in which the terminal hydroxyl groups have been replaced by reactive amine groups or in which the terminal hydroxyl groups have been derivatized to produce an extended chain having terminal amine groups. For example, a some hydrophilic diamine is a "diamino poly(oxyalkylene)" which is poly(alkylene)glycol in which the terminal hydroxyl groups are replaced with amino groups. The term "diamino poly(oxyalkylene" also refers to poly(alkylene)glycols which have aminoalkyl ether groups at the chain termini. One example of a suitable diamino poly(oxyalkylene) is poly(propylene glycol)bis(2-aminopropyl ether). A number of the above polymers can be obtained from Aldrich Chemical Company. Alternatively, conventional methods known in the art can be employed for their synthesis. In some embodiments of the invention, the amount of hydrophilic polymer which is used to make the linear polymer compositions will typically be 10% to 80% by mole relative to the diisocyanate which is used. Typically in these embodiments, the amount is from 20% to 60% by mole relative to the diisocyanate. When lower amounts of hydrophilic polymer are used, it is common to include a chain extender.

Silicone containing polyurethane/polyurea polymers which are useful in the present invention are typically linear, have excellent oxygen permeability and essentially no glucose permeability. Typically, the silicone polymer is a polydimethylsiloxane having two reactive functional groups (i.e., a functionality of 2). The functional groups can be, for example, hydroxyl groups, amino groups or carboxylic acid groups, but are typically hydroxyl or amino groups. In some embodiments, combinations of silicone polymers can be used in which a first portion comprises hydroxyl groups and a second portion comprises amino groups. Typically, the functional groups are positioned at the chain termini of the silicone polymer. A number of suitable silicone polymers are commercially available from such sources as Dow Chemical Company (Midland, Mich., USA) and General Electric Company (Silicones Division, Schenectady, N.Y., USA). Still others can be prepared by general synthetic methods known in the art (see, e.g. U.S. Patent Nos. 5,777,060), beginning with commercially available siloxanes (United Chemical Technologies, Bristol. Pa., USA). For use in the present invention, the silicone polymers will typically be those having a molecular weight of from 400 to 10,000, more typically those having a molecular weight of from 2000 to 4000. The amount of silicone polymer which is incorporated into the reaction mixture will depend on the desired characteristics of the resulting polymer from which the biocompatible membrane are formed. For those compositions in which a lower glucose penetration is desired, a larger amount of silicone polymer can be employed. Alternatively, for compositions in which a higher glucose penetration is desired, smaller amounts of silicone polymer can be employed. Typically, for a glucose sensor, the amount of siloxane polymer will be from 10% to 90% by mole relative to the diisocyanate. Typically, the amount is from 20% to 60% by mole relative to the diisocyanate.
In one group of embodiments, the reaction mixture for the preparation of biocompatible membranes will also contain a chain extender which is an aliphatic or aromatic diol, an aliphatic or aromatic diamine, alkanolamine, or combinations thereof (e.g. as depicted in FIG. 8 of U.S. Patent Nos. 5,777,060)). Examples of suitable aliphatic chain extenders include ethylene glycol, propylene glycol, 1,4-butanediol, 1,6-hexanediol, ethanolamine, ethylene diamine, butane diamine, 1,4-cyclohexanedimethanol. Aromatic chain extenders include, for example, para-di(2-hydroxyethoxy)benzene, meta-di(2-hydroxyethoxy)benzene, Ethacure 100® (a mixture of two isomers of 2,4-diamino-3,5-diethyltoluene), Ethacure 300® (2,4-diamino-3,5-di(methylthio)toluene), 3,3'-dichloro-4,4'diaminodiphenylmethane, Polacure® 740M (trimethylene glycol bis(para-aminobenzoate)ester), and methylenedianiline. Incorporation of one or more of the above chain extenders typically provides the resulting biocompatible membrane with additional physical strength, but does not substantially increase the glucose permeability of the polymer. Typically, a chain extender is used when lower (i.e., 10-40 mol %) amounts of hydrophilic polymers are used. In particularly some compositions, the chain extender is diethylene glycol which is present in from 40% to 60% by mole relative to the diisocyanate.

Polymerization of the above reactants can be carried out in bulk or in a solvent system. Use of a catalyst is some, though not required. Suitable catalysts include dibutyltin bis(2-ethylhexanoate), dibutyltin diacetate, triethylamine and combinations thereof. Typically dibutyltin bis(2-ethylhexanoate is used as the catalyst. Bulk polymerization is typically carried out at an initial temperature of 25° C. (ambient temperature) to 50° C., in order to insure adequate mixing of the reactants. Upon mixing of the reactants, an exotherm is typically observed, with the temperature rising to 90-120° C. After the initial exotherm, the reaction flask can be heated at from 75° C. to 125° C., with 90°. C. to 100 ° C. being a exemplary temperature range. Heating is usually carried out for one to two hours. Solution polymerization can be carried out in a similar manner. Solvents which are suitable for solution polymerization include dimethylformamide, dimethyl sulfoxide, dimethylacetamide, halogenated solvents such as 1,2,3-trichloropropane, and ketones such as 4-methyl-2-pentanone. Typically, THF is used as the solvent. When polymerization is carried out in a solvent, heating of the reaction mixture is typically carried out for three to four hours.

Polymers prepared by bulk polymerization are typically dissolved in dimethylformamide and precipitated from water. Polymers prepared in solvents that are not miscible with water can be isolated by vacuum stripping of the solvent. These polymers are then dissolved in dimethylformamide and precipitated from water. After thoroughly washing with water, the polymers can be dried in vacuo at 50° C. to constant weight.

Preparation of the membranes can be completed by dissolving the dried polymer in a suitable solvent and cast a film onto a glass plate. The selection of a suitable solvent for casting will typically depend on the particular polymer as well as the volatility of the solvent. Typically, the solvent is THF, CHCl₃, CH₂Cl₂, DMF, IPA or combinations thereof. More typically, the solvent is THF or DMF/CH₂ Cl₂ (2/98 volume %). The solvent is removed from the films, the resulting membranes are hydrated fully, their thicknesses measured and water pickup is determined. Membranes which are useful in the present invention will typically have a water pickup of 20 to 100%, typically 30 to 90%, and more typically 40 to 80%, by weight.

Oxygen and glucose diffusion coefficients can also be determined for the individual polymer compositions as well as the stabilized polymeric membranes used according to the present invention. Methods for determining diffusion coefficients are known to those of skill in the art, and examples are provided below. Certain embodiments of the biocompatible membranes described herein will typically have a oxygen diffusion coefficient (Doxygen) of 0.1 x 10⁻⁶ cm² /sec to 2.0 x 10⁻⁶ cm² /sec and a glucose diffusion coefficient (Dglucose) of 1 x 10⁻⁹ cm² /sec to 500 x 10⁻⁹ cm²/sec. More typically, the glucose diffusion coefficient is 10 x 10⁻⁹ cm² /sec to 200 x 10⁻⁹ cm² /sec.

### BRANCHED ACRYLATE POLYMERS

Another polymeric composition used in embodiments of the present invention is a branched acrylate polymer, typically a silicone-based comb-copolymer (see, e.g. U.S. Patent Application Publication No. 2011/0152654). In these compositions, the silicone component typically has very low glass transition temperature (e.g. below room temperature and typically below 0°C) and very high oxygen permeability (e.g. 1 x 10⁻⁷ cm²/sec), characteristics selected to provide advantages such as good mechanical property, higher signal-to-noise ratio, high stability, and highly accurate analysis in in-vivo environments.

Some embodiments of the invention include the use of a composition of matter comprising a blend of different polymers such as a polyurethane/polyurea polymer as discussed above blended with a branched acrylate polymers comprising a hydrophilic comb-copolymer having a central chain and a plurality of side chains coupled to the central chain, wherein at least one side chain comprises a silicone moiety. As is known in the art, a comb-copolymer is one having a structure analogous to a hair comb which has a central backbone to which a plurality of teeth are attached. Such comb-copolymers have a central or main chain (that is roughly analogous to the backbone of the comb) and a plurality of side chains (that are roughly analogous to the teeth of a comb) that branch off of this central chain. This comb-copolymeric structure is shown for example in FIG. 3 of U.S. Patent Application Publication No. 2011/0152654, where the horizontal (-C-CH₂-C-CH₂-C-CH₂-)ₚ portion of the molecule is the central or main chain and the vertical for example (-C-O-C-) portions of the molecule comprise the side chains. These side chains can further have main chain to which various atoms and moieties are attached, for example the vertical (-C-O-C-C-C-Si-O-) side chain shown on the right side of the molecule shown of FIG. 3. For example the horizontal central chain of the side chain shown in this figure has hydrogen and/or methyl atoms and moieties attached thereto. In certain embodiments of the invention, the backbone of at least 1, 2, 3, 4, or 5 different side chains comprises at least 2, 3, 4, 5, 6, 7, 8, 9, 10, 11, 12, 13, 14 or 15 atoms.

The branched acrylate polymers that can be used to make the stabilized polymeric membranes have a number of embodiments. Typically in such embodiments, at least one side chain moiety comprising a silicone moiety comprises a Silicon atom covalently bound to an Oxygen atom (-Si-O-). In some embodiments of the invention, at least one side chain that branches off of the central chain is hydrophilic. In some embodiments of the invention, at least one side chain that branches off of the central chain is hydrophobic. In some embodiments of the invention, at least one side chain that branches off of the central chain is hydrophilic and at least one side chain that branches off of the central chain is hydrophobic. Optionally, the central chain is hydrophilic. Alternatively, the central chain can be hydrophobic, with hydrophilic properties being provided by the side chains.

In certain embodiments of the invention, the central chain comprises a polyvinyl polymer, i.e. a composition formed by polymerizing various vinyl (e.g. CH2=CH-) monomers. Examples include polyvinyl chlorides, polyvinyl acetates, and polyvinyl alcohols. Typically, such polyvinyl polymers comprise polyvinyl acetate, acrylate, acrylamide, acrylonitrile or pyrrolidone subunits. Alternatively the central chain can comprise polyethylene or polypropylene subunits. As is known in the art, such comb copolymers can be made from a variety of different methods, for example a process comprising free radical copolymerization. Typically, the comb-copolymer is made from free radical polymerization of at least one silicone material, and at least one hydrophilic material. Optionally, one or more hydrophobic materials are also used for specific applications and contexts. Illustrative methods and materials for use in making the polymeric compositions of the invention are described for example in U.S. Patent Nos. 6,887,962, 6,809,141, 6,093,781, 5,807,937, 5,708,115, 5,091,480, 5,079,298, 5,061,772, 5,503,461, 6,538,091 and 6,527,850 7,029,688, 7,029,688, 7,001,949; and U.S. Patent Application Nos. 20050143546, 20040024144 and 20030181619, 20040024144. Polymers can be coated onto biosensors using a variety of methods known in the art, for example those described in U.S. Patent Nos. 5,882,494, 6,965,791, 6,934,572, 6,814,845 6,741,877, 6,594,514, 6,477,395, 6,927,246, 5,422,246, 5,286,364, 6,927,033, 5,804,048, 7,003,340, 6,965,791; and U.S. Patent Application Nos. 20060128032, 20060068424, 20050208309, 20040084307, 20030171506, 20030069383, and 20010008931.

Embodiments of the invention include sensors having a membrane comprising the polymeric compositions described herein. An illustrative embodiment is an analyte sensor apparatus for Implantation within a mammal, the analyte sensor apparatus comprising a base layer, a conductive layer disposed upon the base layer wherein the conductive layer includes a working electrode, an analyte sensing layer disposed on the conductive layer, wherein the analyte sensing layer detectably alters the electrical current at the working electrode in the conductive layer in the presence of an analyte, an analyte modulating layer disposed on the analyte sensing layer, wherein the analyte modulating layer modulates the diffusion of the analyte therethrough; the analyte modulating layer comprising a linear polyurethane/polyurea polymer stabilized with a free radical scavenger that can adsorb and bind oxygen. In such analyte sensor apparatus, the membrane having this structure confers a number of desirable properties. Typically for example, the analyte modulating layer has a glucose diffusion coefficient (Dglucose) of from 1 x 10⁻⁹ cm²/sec to 1 x 10⁻⁷ cm²/sec. In addition, typically, the analyte modulating layer has a oxygen diffusion coefficient (Doxygen) to glucose diffusion coefficient (Dglucose) ratio (Doxygen /Dglucose) of 5 to 2000.

### B. TYPICAL COMBINATIONS OF SENSOR ELEMENTS

Embodiments of the invention further include sensors comprising the stabilized polymeric compositions disclosed herein in combination with other sensor elements such as an interference rejection membrane (e.g. an interference rejection membrane as disclosed in U.S. Patent Application Serial Number 12/572,087). In some embodiments of the invention, an element of the sensor apparatus such as an electrode or an aperture is designed to have a specific configuration and/or is mode from a specific material and/or is positioned relative to the other elements so as to facilitate a function of the sensor. In one such embodiment of the invention, a working electrode, a counter electrode and a reference electrode are positionally distributed an the base and/or the conductive layer in a configuration that facilitates sensor start up and/or maintains the hydration of the working electrode, the counter electrode and/or the reference electrode when the sensor apparatus is placed in contact with a fluid comprising the analyte (e.g. by inhibiting shadowing of an electrode, a phenomena which can inhibit hydration and capacitive start-up of a sensor circuit). Typically such embodiments of the invention facilitate sensor start-up and/or initialization.

Optionally embodiments of the apparatus comprise a plurality of working electrodes and/or counter electrodes and/or reference electrodes (e.g. 3 working electrodes, a reference electrode and a counter electrode), in order to, for example, provide redundant sensing capabilities. Certain embodiments of the invention comprising a single sensor. Other embodiments of the invention comprise multiple sensors. In some embodiments of the invention, a pulsed voltage is used to obtain a signal from one or more electrodes of a sensor. Optionally, the plurality of working, counter and reference electrodes are configured together as a unit and positionally distributed on the conductive layer in a repeating pattern of units. In certain embodiments of the invention, the elongated base layer is made from a flexible material that allows the sensor to twist and bend when implanted in vivo; and the electrodes are grouped in a configuration that facilitates an in vivo fluid contacting at least one of working electrode as the sensor apparatus twists and bends when implanted in vivo. In some embodiments, the electrodes are grouped in a configuration that allows the sensor to continue to function if a portion of the sensor having one or more electrodes is dislodged from an in vivo environment and exposed to an ex vivo environment.

In certain embodiments of the invention comprising multiple sensors, elements such as the sensor electrodes are organized/disposed within a flex-circuit assembly. In such embodiments of the invention, the architecture of the sensor system can be designed so that a first sensor does not influence a signal etc. generated by a second sensor (and vice versa); and so that the first and second sensors sense from separate tissue envelopes; so the signals from separate sensors do not interact. At the same time, in typical embodiments of the invention the sensors will be spaced at a distance from each other so that allows them to be easily packaged together and/or adapted to be implanted via a single insertion action. One such embodiment of the invention is an apparatus for monitoring an analyte in a patient, the apparatus comprising: a base element adapted to secure the apparatus to the patient; a first piercing member coupled to and extending from the base element; a first electrochemical sensor operatively coupled to the first piercing member and comprising a first electrochemical sensor electrode for determining at least one physiological characteristic of the patient at a first electrochemical sensor placement site; a second piercing member coupled to and extending from the base element; a second electrochemical sensor operatively coupled to the second piercing member and comprising a second electrochemical sensor electrode for determining at least one physiological characteristic of the patient at a second electrochemical sensor placement site. In such embodiments of the invention, at least one physiological characteristic monitored by the first or the second electrochemical sensor comprises a concentration of a naturally occurring analyte in the patient; the first piercing member disposes the first electrochemical sensor in a first tissue compartment of the patient and the second piercing member disposes the second electrochemical sensor in a second tissue compartment of the patient; and the first and second piercing members are disposed on the base in a configuration selected to avoid a physiological response that can result from implantation of the first electrochemical sensor from altering a sensor signal generated by the second electrochemical sensor.

Various elements of the sensor apparatus can be disposed at a certain location in the apparatus and/or configured in a certain shape and/or be constructed from a specific material so as to facilitate strength and/or function of the sensor. One embodiment of the invention includes an elongated base comprised of a polyimmide or dielectric ceramic material that facilitates the strength and durability of the sensor. In certain embodiments of the invention, the structural features and/or relative position of the working and/or counter and/or reference electrodes is designed to influence sensor manufacture, use and/or function. Optionally, the sensor is operatively coupled to a constellation of elements that comprise a flex-circuit (e.g. electrodes, electrical conduits, contact pads and the like). One embodiment of the invention includes electrodes having one or more rounded edges so as to inhibit delamination of a layer disposed on the electrode (e.g. an analyte sensing layer comprising glucose oxidase). Related embodiments of the invention include methods for inhibiting delamination of a sensor layer using a sensor embodiments of the invention (e.g. one having one or more electrodes having one or more rounded edges). In some embodiments of the invention, a barrier element is disposed on the apparatus so as to inhibit spreading of a layer of material (e.g. an enzyme such as glucose oxidase) disposed on an electrode. Related embodiments of the invention include methods for inhibiting movement of a compound disposed on a sensor embodiments of the invention (e.g. one constructed to have such a barrier structure). Optionally, a barrier element is disposed on the apparatus so as to encircle a reactive surface of an electrode.

In typical embodiments of the invention, the sensor is operatively coupled to further elements (e.g. electronic components) such as elements designed to transmit and/or receive a signal, monitors, processors and the like as well as devices that can use sensor data to modulate a patient's physiology such as medication infusion pumps. For example, in some embodiments of the invention, the sensor is operatively coupled to a sensor input capable of receiving a signal from the sensor that is based on a sensed physiological characteristic value in the mammal; and a processor coupled to the sensor input, wherein the processor is capable of characterizing one or more signals received from the sensor. A wide variety of sensor configurations as disclosed herein can be used in such systems. Optionally, for example, the sensor comprises three working electrodes, one counter electrode and one reference electrode. In certain embodiments, at least one working electrode is coated with an analyte sensing layer comprising glucose oxidase and at least one working electrode is not coated with an analyte sensing layer comprising glucose oxidase.

### C. DIAGRAMMATIC ILLUSTRATION OF TYPICAL SENSOR CONFIGURATIONS

FIG. 2A illustrates a cross-section of a typical sensor embodiment 100 of the present invention. This sensor embodiment is formed from a plurality of components that are typically in the form of layers of various conductive and non-conductive constituents disposed on each other according to art accepted methods and/or the specific methods of the invention disclosed herein. The components of the sensor are typically characterized herein as layers because, for example, it allows for a facile characterization of the sensor structure shown in FIG. 2. Artisans will understand however, that in certain embodiments of the invention, the sensor constituents are combined such that multiple constituents form one or more heterogeneous layers. In this context, those of skill in the art understand that the ordering of the layered constituents can be altered in various embodiments of the invention.

The embodiment shown in Figure 2A includes a base layer 102 to support the sensor 100. The base layer 102 can be made of a material such as a metal and/or a ceramic and/or a polymeric substrate, which may be self-supporting or further supported by another material as is known in the art. Embodiments of the invention include a conductive layer 104 which is disposed on and/or combined with the base layer 102. Typically the conductive layer 104 comprises one or more electrodes. An operating sensor 100 typically includes a plurality of electrodes such as a working electrode, a counter electrode and a reference electrode. Other embodiments may also include a plurality of working and/or counter and/or reference electrodes and/or one or more electrodes that performs multiple functions, for example one that functions as both as a reference and a counter electrode.

As discussed in detail below, the base layer 102 and/or conductive layer 104 can be generated using many known techniques and materials. In certain embodiments of the invention, the electrical circuit of the sensor is defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating cover layer 106 such as a polymer coating can be disposed on portions of the sensor 100. Acceptable polymer coatings for use as the insulating protective cover layer 106 can include, but are not limited to, non-toxic biocompatible polymers such as silicone compounds, polyimides, biocompatible solder masks, epoxy acrylate copolymers, or the like. In the sensors of the present invention, one or more exposed regions or apertures 108 can be made through the cover layer 106 to open the conductive layer 104 to the external environment and to, for example, allow an analyte such as glucose to permeate the layers of the sensor and be sensed by the sensing elements. Apertures 108 can be formed by a number of techniques, including laser ablation, tape masking, chemical milling or etching or photolithographic development or the like. In certain embodiments of the invention, during manufacture, a secondary photoresist can also be applied to the protective layer 106 to define the regions of the protective layer to be removed to form the aperture(s) 108. The exposed electrodes and/or contact pads can also undergo secondary processing (e.g. through the apertures 108), such as additional plating processing, to prepare the surfaces and/or strengthen the conductive regions.

In the sensor configuration shown in Figure 2A, an analyte sensing layer 110 (which is typically a sensor chemistry layer, meaning that materials in this layer undergo a chemical reaction to produce a signal that can be sensed by the conductive layer) is disposed on one or more of the exposed electrodes of the conductive layer 104. In the sensor configuration shown in Figure 2B, an interference rejection membrane 120 is disposed on one or more of the exposed electrodes of the conductive layer 104, with the analyte sensing layer 110 then being disposed on this interference rejection membrane 120. Typically, the analyte sensing layer 110 is an enzyme layer. Most typically, the analyte sensing layer 110 comprises an enzyme capable of producing and/or utilizing oxygen and/or hydrogen peroxide, for example the enzyme glucose oxidase. Optionally the enzyme in the analyte sensing layer is combined with a second carrier protein such as human serum albumin, bovine serum albumin or the like. In an illustrative embodiment,
an oxidoreductase enzyme such as glucose oxidase in the analyte sensing layer 110 reacts with glucose to produce hydrogen peroxide, a compound which then modulates a current at an electrode. As this modulation of current depends on the concentration of hydrogen peroxide, and the concentration of hydrogen peroxide correlates to the concentration of glucose, the concentration of glucose can be determined by monitoring
this modulation in the current. In a specific embodiment of the invention, the hydrogen peroxide is oxidized at a working electrode which is an anode (also termed herein the anodic working electrode), with the resulting current being proportional to the hydrogen peroxide concentration. Such modulations in the current caused by changing hydrogen peroxide concentrations can by monitored by any one of a variety of sensor detector apparatuses such as a universal sensor amperometric biosensor detector or one of the other variety of similar devices known in the art such as glucose monitoring devices produced by Medtronic MiniMed.

In embodiments of the invention, the analyte sensing layer 110 can be applied over portions of the conductive layer or over the entire region of the conductive layer.
Typically the analyte sensing layer 110 is disposed on the working electrode which can be the anode or the cathode. Optionally, the analyte sensing layer 110 is also disposed on a counter and/or reference electrode. While the analyte sensing layer 110 can be up to 1000 microns (i.tm) in thickness, typically the analyte sensing layer is relatively thin as compared to those found in sensors previously described in the art, and is for example, typically less than 1, 0.5, 0.25 or 0.1 microns in thickness. As discussed in detail below, some methods for generating a thin analyte sensing layer 110 include brushing the layer onto a substrate (e.g. the reactive surface of a platinum black electrode), as well as spin coating processes, dip and dry processes, low shear spraying processes, ink-jet printing processes, silk screen processes.

Typically, the analyte sensing layer 110 is coated and or disposed next to one or more additional layers. Optionally, the one or more additional layers includes a protein layer 116 disposed upon the analyte sensing layer 110. Typically, the protein layer 116 comprises a protein such as human serum albumin, bovine serum albumin or the like. Typically, the protein layer 116 comprises human serum albumin. In some embodiments of the invention, an additional layer includes an analyte modulating layer 112 that is disposed above the analyte sensing layer 110 to regulate analyte access with the analyte sensing layer 110. For example, the analyte modulating membrane layer 112 can comprise a glucose limiting membrane, which regulates the amount of glucose that contacts an enzyme such as glucose oxidase that is present in the analyte sensing layer.
Such glucose limiting membranes can be made from a wide variety of materials known to be suitable for such purposes, e.g., silicone compounds such as polydimethyl siloxanes, polyurethanes, polyurea cellulose acetates, NAFION, polyester sulfonic acids (e.g. Kodak AQ), hydrogels, the polymer blends disclosed herein or any other suitable hydrophilic membranes known to those skilled in the art.

In some embodiments of the invention, an adhesion promoter layer 114 is disposed between layers such as the analyte modulating layer 112 and the analyte sensing layer 110 as shown in FIG. 2 in order to facilitate their contact and/or adhesion. In a specific embodiment of the invention, an adhesion promoter layer 114 is disposed between the analyte modulating layer 112 and the protein layer 116 as shown in FIG. 2 in
order to facilitate their contact and/or adhesion. The adhesion promoter layer 114 can be made from any one of a wide variety of materials known in the art to facilitate the bonding between such layers. Typically, the adhesion promoter layer 114 comprises a silane compound. In alternative embodiments, protein or like molecules in the analyte sensing layer 110 can be sufficiently crosslinked or otherwise prepared to allow the
analyte modulating membrane layer 112 to be disposed in direct contact with the analyte sensing layer 110 in the absence of an adhesion promoter layer 114.

Embodiments of typical elements used to make the sensors disclosed herein are discussed below.

### D. TYPICAL ANALYTE SENSOR CONSTITUENTS USED IN EMBODIMENTS OF THE INVENTION

The following disclosure provides examples of typical elements/constituents used in sensor embodiments of the invention. While these elements can be described as discreet units (e.g. layers), those of skill in the art understand that sensors can be designed to contain elements having a combination of some or all of the material properties and/or functions of the elements/constituents discussed below (e.g. an element that serves both as a supporting base constituent and/or a conductive constituent and/or a matrix for the analyte sensing constituent and which further functions as an electrode in the sensor). Those in the art understand that these thin film analyte sensors can be adapted for use in a number of sensor systems such as those described below.

### BASE CONSTITUENT

Sensors of the invention typically include a base constituent (see, e.g. element 102 in Figure 2A). The term "base constituent" is used herein according to art accepted terminology and refers to the constituent in the apparatus that typically provides a supporting matrix for the plurality of constituents that are stacked on top of one another and comprise the functioning sensor. In one form, the base constituent comprises a thin film sheet of insulative (e.g. electrically insulative and/or water impermeable) material. This base constituent can be made of a wide variety of materials having desirable qualities such as dielectric properties, water impermeability and hermeticity. Some materials include metallic, and/or ceramic and/or polymeric substrates or the like.

The base constituent may be self-supporting or further supported by another material as is known in the art. In one embodiment of the sensor configuration shown in Figure 2A, the base constituent 102 comprises a ceramic. Alternatively, the base constituent comprises a polymeric material such as a polyimide. In an illustrative embodiment, the ceramic base comprises a composition that is predominantly Al₂O₃ (e.g. 96%). The use of alumina as an insulating base constituent for use with implantable devices is disclosed in U.S. Pat. Nos. 4,940,858, 4,678,868 and 6,472,122. The base constituents of the invention can further include other elements known in the art, for example hermetical vias (see, e.g. WO 03/023388). Depending upon the specific sensor design, the base constituent can be relatively thick constituent (e.g. thicker than 50, 100, 200, 300, 400, 500 or 1000 microns). Alternatively, one can utilize a nonconductive ceramic, such as alumina, in thin constituents, e.g., less than 30 microns.

### CONDUCTIVE CONSTITUENT

The electrochemical sensors of the invention typically include a conductive constituent disposed upon the base constituent that includes at least one electrode for measuring an analyte or its byproduct (e.g. oxygen and/or hydrogen peroxide) to be assayed (see, e.g. element 104 in Figure 2A). The term "conductive constituent" is used herein according to art accepted terminology and refers to electrically conductive sensor elements such as electrodes which are capable of measuring and a detectable signal and conducting this to a detection apparatus. An illustrative example of this is a conductive constituent that can measure an increase or decrease in current in response to exposure to a stimuli such as the change in the concentration of an analyte or its byproduct as compared to a reference electrode that does not experience the change in the concentration of the analyte, a coreactant (e.g. oxygen) used when the analyte interacts with a composition (e.g. the enzyme glucose oxidase) present in analyte sensing constituent 110 or a reaction product of this interaction (e.g. hydrogen peroxide). Illustrative examples of such elements include electrodes which are capable of producing variable detectable signals in the presence of variable concentrations of molecules such as hydrogen peroxide or oxygen. Typically one of these electrodes in the conductive constituent is a working electrode, which can be made from non-corroding metal or carbon. A carbon working electrode may be vitreous or graphitic and can be made from a solid or a paste. A metallic working electrode may be made from platinum group metals, including palladium or gold, or a non-corroding metallically conducting oxide, such as ruthenium dioxide. Alternatively the electrode may comprise a silver/silver chloride electrode composition. The working electrode may be a wire or a thin conducting film applied to a substrate, for example, by coating or printing. Typically, only a portion of the surface of the metallic or carbon conductor is in electrolytic contact with the analyte-containing solution. This portion is called the working surface of the electrode. The remaining surface of the electrode is typically isolated from the solution by an electrically insulating cover constituent 106. Examples of useful materials for generating this protective cover constituent 106 include polymers such as polyimides, polytetrafluoroethylene, polyhexafluoropropylene and silicones such as polysiloxanes.

In addition to the working electrode, the analyte sensors of the invention typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors of the present invention have one or more working electrodes and one or more counter, reference, and/or counter/reference electrodes. One embodiment of the sensor of the present invention has two, three or four or more working electrodes. These working electrodes in the sensor may be integrally connected or they may be kept separate.

Typically for in vivo use, embodiments of the present invention are implanted subcutaneously in the skin of a mammal for direct contact with the body fluids of the mammal, such as blood. Alternatively the sensors can be implanted into other regions within the body of a mammal such as in the intraperotineal space. When multiple working electrodes are used, they may be implanted together or at different positions in the body. The counter, reference, and/or counter/reference electrodes may also be implanted either proximate to the working electrode(s) or at other positions within the body of the mammal.

### INTERFERENCE REJECTION CONSTITUENT

The electrochemical sensors of the Invention optionally include an interference rejection constituent disposed between the surface of the electrode and the environment to be assayed. In particular, certain sensor embodiments rely on the oxidation and/or reduction of hydrogen peroxide generated by enzymatic reactions on the surface of a working electrode at a constant potential applied. Because amperometric detection based on direct oxidation of hydrogen peroxide requires a relatively high oxidation potential, sensors employing this detection scheme may suffer interference from oxidizable species that are present in biological fluids such as ascorbic acid, uric acid and acetaminophen. In this context, the term "interference rejection constituent" is used herein according to art accepted terminology and refers to a coating or membrane in the sensor that functions to inhibit spurious signals generated by such oxidizable species which interfere with the detection of the Signal generated by the analyte to be sensed. Certain interference rejection constituents function via size exclusion (e.g. by excluding interfering species of a specific size). Examples of interference rejection constituents include one or more layers or coatings of compounds such as hydrophilic crosslinked pHEMA and polylysine polymers as well as cellulose acetate (including cellulose acetate incorporating agents such as poly(ethylene glycol)), polyethersulfones, polytetra-fluoroethylenes, the perfluoronated ionomer NAFION, polyphenylenediamine, epoxy Illustrative discussions of such interference rejection constituents are found for example in Ward et al., Biosensors and Bioelectronics 17 (2002) 181-189 and Choi et al., Analytical Chimica Acta 461 (2002) 251-260. Other interference rejection constituents include for example those observed to limit the movement of compounds based upon a molecular weight range, for example cellulose acetate as disclosed for example in U.S. Patent No. 5,755,939. Additional compositions having an unexpected constellation of material properties that make them ideal for use as interference rejection membranes in certain amperometric glucose sensors as well as methods for making and using them are disclosed herein, for example in U.S. Patent Application Serial Number 12/572,087.

### ANALYTE SENSING CONSTITUENT

The electrochemical sensors of the invention include an analyte sensing constituent disposed on the electrodes of the sensor (see, e.g. element 110 in Figure 2A). The term "analyte sensing constituent" is used herein according to art accepted terminology and refers to a constituent comprising a material that is capable of recognizing or reacting with an analyte whose presence is to be detected by the analyte sensor apparatus. Typically this material in the analyte sensing constituent produces a detectable signal after interacting with the analyte to be sensed, typically via the electrodes of the conductive constituent. In this regard the analyte sensing constituent and the electrodes of the conductive constituent work in combination to produce the electrical signal that is read by an apparatus associated with the analyte sensor. Typically, the analyte sensing constituent comprises an oxidoreductase enzyme capable of reacting with and/or producing a molecule whose change in concentration can be measured by measuring the change in the current at an electrode of the conductive constituent (e.g. oxygen and/or hydrogen peroxide), for example the enzyme glucose oxidase. An enzyme capable of producing a molecule such as hydrogen peroxide can be disposed on the electrodes according to a number of processes known in the art. The analyte sensing constituent can coat all or a portion of the various electrodes of the sensor. In this context, the analyte sensing constituent may coat the electrodes to an equivalent degree. Alternatively the analyte sensing constituent may coat different electrodes to different degrees, with for example the coated surface of the working electrode being larger than the coated surface of the counter and/or reference electrode.

Typical sensor embodiments of this element of the invention utilize an enzyme (e.g. glucose oxidase) that has been combined with a second protein (e.g. albumin) in a fixed ratio (e.g. one that is typically optimized for glucose oxidase stabilizing properties) and then applied on the surface of an electrode to form a thin enzyme constituent. In a typical embodiment, the analyte sensing constituent comprises a GOx and HSA mixture. In a typical embodiment of an analyte sensing constituent having GOx, the GOx reacts with glucose present in the sensing environment (e.g. the body of a mammal) and generates hydrogen peroxide according to the reaction shown in Figure 1, wherein the hydrogen peroxide so generated is anodically detected at the working electrode in the conductive constituent.

As noted above, the enzyme and the second protein (e.g. an albumin) are typically treated to form a crosslinked matrix (e.g. by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. Patent Application Serial Number 10/335,506 and PCT publication WO 03/035891. For example, an amine cross-linking reagent, such as, but not limited to, glutaraldehyde, can be added to the protein mixture.

### PROTEIN CONSTITUENT

The electrochemical sensors of the invention optionally include a protein constituent disposed between the analyte sensing constituent and the analyte modulating constituent (see, e.g. element 116 in Figure 2A). The term "protein constituent" is used herein according to art accepted terminology and refers to constituent containing a carrier protein or the like that is selected for compatibility with the analyte sensing constituent and/or the analyte modulating constituent. In typical embodiments, the protein constituent comprises an albumin such as human serum albumin. The HSA concentration may vary between 0.5%-30% (w/v). Typically the HSA concentration is 1-10% w/v, and most typically is 5% w/v. In alternative embodiments of the invention, collagen or BSA or other structural proteins used in these contexts can be used instead of or in addition to HSA. This constituent is typically crosslinked on the analyte sensing constituent according to art accepted protocols.

### ADHESION PROMOTING CONSTITUENT

The electrochemical sensors of the invention can include one or more adhesion promoting (AP) constituents (see, e.g. element 114 in Figure 2A). The term "adhesion promoting constituent" is used herein according to art accepted terminology and refers to a constituent that includes materials selected for their ability to promote adhesion between adjoining constituents in the sensor. Typically, the adhesion promoting constituent is disposed between the analyte sensing constituent and the analyte modulating constituent. Typically, the adhesion promoting constituent is disposed between the optional protein constituent and the analyte modulating constituent. The adhesion promoter constituent can be made from any one of a wide variety of materials known in the art to facilitate the Bonding between such constituents and can be applied by any one of a wide variety of methods known in the art. Typically, the adhesion promoter constituent comprises a silane compound such as γ-aminopropyltrimethoxysilane.

The use of silane coupling reagents, especially those of the formula R'Si(OR)₃ in which R' is typically an aliphatic group with a terminal amine and R is a lower alkyl group, to promote adhesion is known in the art (see, e.g. U.S. Patent No. 5,212,050). For example, chemically modified electrodes in which a silane such as γ-aminopropyltriethoxysilane and glutaraldehyde were used in a step-wise process to attach and to co-crosslink bovine serum albumin (BSA) and glucose oxidase (GOx) to the electrode surface are well known in the art (see, e.g. Yao, T. Analytica Chim. Acta 1983, 148, 27-33).

In certain embodiments of the invention, the adhesion promoting constituent further comprises one or more compounds that can also be present in an adjacent constituent such as the polydimethyl siloxane (PDMS) compounds that serv es to limit the diffusion of analytes such as glucose through the analyte modulating constituent. In illustrative embodiments the formulation comprises 0.5-20% PDMS, typically 5-15% PDMS, and most typically 10% PDMS. In certain embodiments of the invention, the adhesion promoting constituent is crosslinked within the layered sensor system and correspondingly includes an agent selected for its ability to crosslink a moiety present in a proximal constituent such as the analyte modulating constituent. In illustrative embodiments of the invention, the adhesion promoting constituent includes an agent selected for its ability to crosslink an amine or carboxyl moiety of a protein present in a proximal constituent such as the analyte sensing constituent and/or the protein constituent and or a siloxane moiety present in a compound disposed in a proximal layer such as the analyte modulating layer.

### ANALYTE MODULATING CONSTITUENT

The electrochemical sensors of the invention include an analyte modulating constituent disposed on the sensor (see, e.g. element 112 in Figure 2A). The term "analyte modulating constituent" is used herein according to art accepted terminology and refers to a constituent that typically forms a membrane on the sensor that operates to modulate the diffusion of one or more analytes, such as glucose, through the constituent. In certain embodiments of the invention, the analyte modulating constituent is an analyte-limiting membrane operates to prevent or restrict the diffusion of one or more analytes, such as glucose, through the constituents. (e.g. a stabilized glucose limiting membrane as shown in FIG. 4A and 4B) which In other embodiments of the invention, the analyte-modulating constituent operates to facilitate the diffusion of one or more analytes, through the constituents. Optionally such analyte modulating constituents can be formed to prevent or restrict the diffusion of one type of molecule through the constituent (e.g. glucose), while at the same time allowing or even facilitating the diffusion of other types of molecules through the constituent (e.g. O₂).

With respect to glucose sensors, in known enzyme electrodes, glucose and oxygen from blood, as well as some interferents, such as ascorbic acid and uric acid, diffuse through a primary membrane of the sensor. As the glucose, oxygen and interferents reach the analyte sensing constituent, an enzyme, such as glucose oxidase, catalyzes the conversion of glucose to hydrogen peroxide and gluconolactone. The hydrogen peroxide may diffuse back through the analyte modulating constituent, or it may diffuse to an electrode where it can be reacted to form oxygen and a proton to produce a current that is proportional to the glucose concentration. The sensor membrane assembly serves several functions, including selectively allowing the passage of glucose therethrough. In this context, an illustrative analyte modulating constituent is a semi-permeable membrane which permits passage of water, oxygen and at least one selective analyte and which has the ability to absorb water, the membrane having a water soluble, hydrophilic polymer.

A variety of illustrative analyte modulating compositions are known in the art and are described for example in U.S. Patent Nos. 6,319,540, 5,882,494, 5,786,439 5,777,060, 5,771,868 and 5,391,250.
The hydrogels described therein are particularly useful with a variety of implantable devices for which it is advantageous to provide a surrounding water constituent. In typical embodiments of the invention, the analyte modulating composition comprises the stabilized polymeric compositions disclosed herein (e.g. the embodiments of this composition that are shown in FIG. 3).
In one illustrative embodiment of the invention, the analyte modulating layer comprises a polyurethane/polyurea polymer formed from a mixture comprising: a diisocyanate; a hydrophilic polymer comprising a hydrophilic diol or hydrophilic diamine; a siloxane having an amino, hydroxyl or carboxylic acid functional group; and a stabilizing agent as disclosed herein (e.g. pyrogallol). Optionally, this stabilized polymer is blended with a branched acrylate polymer formed from a mixture comprising: a butyl, propyl, ethyl or methyl-acrylate; an amino-acrylate; a siloxane-acrylate; and a poly(ethylene oxide)-acrylate. Optionally, additional materials can be included in these polymeric blends. For example, certain embodiments of the branched acrylate polymer are formed from a reaction mixture that includes a hydroxyl-acrylate compound (e.g. 2-hydroxyethyl methacrylate).

In a specific embodiment of the invention, the analyte modulating layer comprises a polyurethane/polyurea polymer formed from a mixture comprising a diisocyanate; a hydrophilic polymer comprising a hydrophilic diol or hydrophilic diamine; and a siloxane having an amino, hydroxyl or carboxylic acid functional group at a terminus, with this polyurethane/polyurea polymer being stabilized with a branched acrylate polymer formed from a mixture comprising a methyl methacrylate; a 2-(dimethylamino)ethyl methacrylate; a polydimethyl siloxane monomethacryloxypropyl; a poly(ethylene oxide) methyl ether methacrylate; and a 2-hydroxyethyl methacrylate. Typically, the first polymer is formed from a mixture comprising: a diisocyanate compound (typically 50 mol% of the reactants in the mixture); at least one hydrophilic diol or hydrophilic diamine compound (typically 17 to 45 mol% of the reactants in the mixture); and a siloxane compound. Optionally the first polyurethane/polyurea polymer comprises 45-55 mol% (e.g. 50 mol%) of a diisocyanate (e.g. 4,4'-diisocyanate), 10-20 (e.g. 12.5 mol%) mol% of a siloxane (e.g. polymethylhydrosiloxane, trimethylsilyl terminated), 30-45 mol% (e.g. 37.5 mol%) of a hydrophilic diol or hydrophilic diamine compound (e.g. polypropylene glycol diamine having an average molecular weight of 600 Daltons, Jeffamine 600) and 0.1-5 weight % of a polyurethane/polyurea polymer stabilizing compound as disclosed herein. This first polyurethane/polyurea polymer is optionally mixed with a second polymer formed from a mixture comprising: 5-45 weight % of a 2-(dimethylamino)ethyl methacrylate compound; 15-55 weight % of a methyl methacrylate compound; 15-55 weight % of a polydimethyl siloxane monomethacryloxypropyl compound; 5-35 weight % of a poly(ethylene oxide) methyl ether methacrylate compound; and 1-20 weight % 2-hydroxyethyl methacrylate, with the first polymer and the second polymer stabilized together at a ratio between 1:1 and 1:20 weight %.

### COVER CONSTITUENT

The electrochemical sensors of the Invention include one or more cover constituents which are typically electrically insulating protective constituents (see, e.g. element 106 in Figure 2A). Typically, such cover constituents can be in the form of a coating, sheath or tube and are disposed on at least a portion of the analyte modulating constituent. Acceptable polymer coatings for use as the insulating protective cover constituent can include, but are not limited to, non-toxic biocompatible polymers such as silicone compounds, polyimides, biocompatible solder masks, epoxy acrylate copolymers,
or the like. Further, these coatings can be photo-imageable to facilitate photolithographic forming of apertures through to the conductive constituent. A typical cover constituent comprises spun on silicone. As is known in the art, this constituent can be a commercially available RTV (room temperature vulcanized) silicone composition. A typical chemistry in this context is polydimethyl siloxane (acetoxy based).

### E. ILLUSTRATIVE EMBODIMENTS OF ANALYTE SENSOR APPARATUS AND ASSOCIATED CHARACTERISTICS

The analyte sensor apparatus disclosed herein has a number of embodiments. A general embodiment of the invention is an analyte sensor apparatus for implantation within a mammal. While the analyte sensors are typically designed to be implantable within the Body of a mammal, the sensors are not limited to any particular environment and can instead be used in a wide variety of contexts, for example for the analysis of most liquid samples including biological fluids such as whole-blood, lymph, plasma, serum, saliva, urine, stool, perspiration, mucus, tears, cerebrospinal fluid, nasal secretion, cervical or vaginal secretion, semen, pleural fluid, amniotic fluid, peritoneal fluid, middle ear fluid, joint fluid, gastric aspirate or the like. In addition, solid or desiccated samples may be dissolved in an appropriate solvent to provide a liquid mixture suitable for analysis.

As noted above, the sensor embodiments disclosed herein can be used to sense analytes of interest in one or more physiological environments. In certain embodiments for example, the sensor can be in direct contact with interstitial fluids as typically occurs with subcutaneous sensors. The sensors of the present invention may also be part of a skin surface system where interstitial glucose is extracted through the skin and brought into contact with the sensor (see, e.g. U.S. Patent Nos. 6,155,992 and 6,706,159). In other embodiments, the sensor can be in contact with blood as typically occurs for example with intravenous sensors. The sensor embodiments of the invention further include those adapted for use in a variety of contexts. In certain embodiments for example, the sensor can be designed for use in mobile contexts, such as those employed by ambulatory users. Alternatively, the sensor can be designed for use in stationary contexts such as those adapted for use in clinical settings. Such sensor embodiments include, for example, those used to monitor one or more analytes present in one or more physiological environments in a hospitalized patient.
Sensors of the invention can also be incorporated in to a wide variety of medical systems known in the art. Sensors of the invention can be used, for example, in a closed loop infusion systems designed to control the rate that medication is infused into the body of a user. Such a closed loop infusion system can include a sensor and an associated meter which generates an input to a controller which in turn operates a delivery system (e.g. one that calculates a dose to be delivered by a medication infusion pump). In such contexts, the meter associated with the sensor may also transmit commands to, and be used to remotely control, the delivery system. Typically, the sensor is a subcutaneous sensor in contact with interstitial fluid to monitor the glucose concentration in the body of the user, and the liquid infused by the delivery system into the body of the user includes insulin. Illustrative systems are disclosed for example in U. S. Patent Nos. 6,558,351 and 6,551,276; PCT Application Nos. US99/21703 and US99/22993; as well as WO 2004/008956 and WO 2004/009161.

### F. ANALYTE SENSOR APPARATUS CONFIGURATIONS

In a clinical setting, accurate and relatively fast determinations of analytes such as glucose and/or lactate levels can be determined from blood samples utilizing electrochemical sensors. Conventional sensors are fabricated to be large, comprising many serviceable parts, or small, planar-type sensors which may be more convenient in many circumstances. The term "planar" as used herein refers to the well-known procedure of fabricating a substantially planar structure comprising layers of relatively thin materials, for example, using the well-known thick or thin-film techniques. See, for example, Liu et al., U.S. Pat. No. 4,571,292, and Papadakis et al., U.S. Pat. No. 4,536,274. As noted below, embodiments of the invention disclosed herein have a wider range of geometrical configurations (e.g. planar) than existing sensors in the art. In addition, certain embodiments of the invention include one or more of the sensors disclosed herein coupled to another apparatus such as a medication infusion pump.

Figure 2 provides a diagrammatic view of a typical analyte sensor configuration of the current invention. Certain sensor configurations are of a relatively flat "ribbon" type configuration that can be made with the analyte sensor apparatus. Such "ribbon" type configurations illustrate an advantage of the sensors disclosed herein that arises due to the spin coating of sensing enzymes such as glucose oxidase, a manufacturing step that produces extremely thin enzyme coatings that allow for the design and production of highly flexible sensor geometries. Such thin enzyme coated sensors provide further advantages such as allowing for a smaller sensor area while maintaining sensor sensitivity, a highly desirable feature for implantable devices (e.g. smaller devices are easier to implant). Consequently, sensor embodiments of the invention that utilize very thin analyte sensing layers that can be formed by processes such as spin coating can have a wider range of geometrical configurations (e.g. planar) than those sensors that utilize enzyme layers formed via processes such as electrodeposition.

Certain sensor configurations include multiple conductive elements such as multiple working, counter and reference electrodes. Advantages of such configurations include increased surface area which provides for greater sensor sensitivity. For example, one sensor configuration introduces a third working sensor. One obvious advantage of such a configuration is signal averaging of three sensors which increases sensor accuracy. Other advantages include the ability to measure multiple analytes. In particular, analyte sensor configurations that include electrodes in this arrangement (e.g. multiple working, counter and reference electrodes) can be incorporated into multiple analyte sensors. The measurement of multiple analytes such as oxygen, hydrogen peroxide, glucose, lactate, potassium, calcium, and any other physiologically relevant substance/analyte provides a number of advantages, for example the ability of such sensors to provide a linear response as well as ease in calibration and/or recalibration.

An exemplary multiple sensor device comprises a single device having a first sensor which is polarized cathodically and designed to measure the changes in oxygen concentration that occur at the working electrode (a cathode) as a result of glucose interacting with glucose oxidase; and a second sensor which is polarized anodically and designed to measure changes in hydrogen peroxide concentration that occurs at the working electrode (an anode) as a result of glucose coming form the external environment and interacting with glucose oxidase. As is known in the art, in such designs, the first oxygen sensor will typically experience a decrease in current at the working electrode as oxygen contacts the sensor while the second hydrogen peroxide sensor will typically experience an increase in current at the working electrode as the hydrogen peroxide generated as shown in Figure 1 contacts the sensor. In addition, as is known in the art, an observation of the change in current that occurs at the working electrodes as compared to the reference electrodes in the respective sensor systems correlates to the change in concentration of the oxygen and hydrogen peroxide molecules which can then be correlated to the concentration of the glucose in the external environment (e.g. the body of the mammal).

The analyte sensors of the invention can be coupled with other medical devices such as medication infusion pumps. In an illustrative variation of this scheme, replaceable analyte sensors of the invention can be coupled with other medical devices such as medication infusion pumps, for example by the use of a port couple to the medical device (e.g. a subcutaneous port with a locking electrical connection).

### II. ILLUSTRATIVE METHODS AND MATERIALS FOR MAKING ANALYTE SENSOR APPARATUS OF THE INVENTION

A number of articles, U.S. patents and patent application describe the state of the art with the common methods and materials disclosed herein and further describe various elements (and methods for their manufacture) that can be used in the sensor designs disclosed herein. These include for example, U.S. Patent Nos. 6,413,393; 6,368,274; 5,786,439; 5,777,060; 5,391,250; 5,390,671; 5,165,407, 4,890,620, 5,390,671, 5,390,691, 5,391,250, 5,482,473, 5,299,571, 5,568,806; United States Patent Application 20020090738; as well as PCT International Publication Numbers WO 01/58348, WO 03/034902, WO 03/035117, WO 03/035891, WO 03/023388, WO 03/022128, WO 03/022352, WO 03/023708, WO 03/036255, WO03/036310 and WO 03/074107.

Typical sensors for monitoring glucose concentration of diabetics are further described in Shichiri, et al.,: "In Vivo Characteristics of Needle-Type Glucose Sensor-Measurements of Subcutaneous Glucose Concentrations in Human Volunteers," Horm. Metab. Res., Suppl. Ser. 20:17-20 (1988); Bruckel, et al.,: "In Vivo Measurement of Subcutaneous Glucose Concentrations with an Enzymatic Glucose Sensor and a Wick Method," Klin. Wochenschr. 67:491-495 (1989); and Pickup, et al.,: "In Vivo Molecular Sensing in Diabetes Mellitus: An Implantable Glucose Sensor with Direct Electron Transfer," Diabetologia 32:213-217 (1989). Other sensors are described in, for example Reach, et al., in ADVANCES IN IMPLANTABLE DEVICES, A. Turner (ed.), JAI Press, London, Chap. 1, (1993).
A typical embodiment of the invention disclosed herein is a method of making a sensor apparatus for Implantation within a mammal comprising the steps of: providing a base layer; forming a conductive layer on the base layer, wherein the conductive layer includes an electrode (and typically a working electrode, a reference electrode and a counter electrode); forming an analyte sensing layer on the conductive layer, wherein the analyte sensing layer includes a composition that can alter the electrical current at the electrode in the conductive layer in the presence of an analyte; optionally forming a protein layer on the analyte sensing layer; forming an adhesion promoting layer on the analyte sensing layer or the optional protein layer; forming an analyte modulating layer disposed on the adhesion promoting layer, wherein the analyte modulating layer includes a composition that modulates the diffusion of the analyte therethrough; and forming a cover layer disposed on at least a portion of the analyte modulating layer, wherein the cover layer further includes an aperture over at least a portion of the analyte modulating layer. In certain embodiments of the invention, the analyte modulating layer comprises a linear polyurethane/polyurea polymer stabilized with a branched acrylate copolymer having a central chain and a plurality of side chains coupled to the central chain. In some embodiments of these methods, the analyte sensor apparatus is formed in a planar geometric configuration

As disclosed herein, the various layers of the sensor can be manufactured to exhibit a variety of different characteristics which can be manipulated according to the specific design of the sensor. For example, the adhesion promoting layer includes a compound selected for its ability to stabilize the overall sensor structure, typically a silane composition. In some embodiments of the invention, the analyte sensing layer is formed by a spin coating process and is of a thickness selected from the group consisting of less than 1, 0.5, 0.25 and 0.1 microns in height.

Typically, a method of making the sensor includes the step of forming a protein layer on the analyte sensing layer, wherein a protein within the protein layer is an albumin selected from the group consisting of bovine serum albumin and human serum albumin. Typically, a method of making the sensor includes the step of forming an analyte sensing layer that comprises an enzyme composition selected from the group consisting of glucose oxidase, glucose dehydrogenase, lactate oxidase, hexokinase and lactate dehydrogenase. In such methods, the analyte sensing layer typically comprises a carrier protein composition in a substantially fixed ratio with the enzyme, and the enzyme and the carrier protein are distributed in a substantially uniform manner throughout the analyte sensing layer.
The disclosure provided herein includes sensors and sensor designs that can be generated using combinations of various well known techniques. The disclosure further provides methods for applying very thin enzyme coatings to these types of sensors as well as sensors produced by such processes. In this context, some embodiments of the invention include methods for making such sensors on a substrate according to art accepted processes. In certain embodiments, the substrate comprises a rigid and flat structure suitable for use in photolithographic mask and etch processes. In this regard, the substrate typically defines an upper surface having a high degree of uniform flatness. A polished glass plate may be used to define the smooth upper surface. Alternative substrate materials include, for example, stainless steel, aluminum, and plastic materials such as delrin, etc. In other embodiments, the substrate is non-rigid and can be another layer of Film or insulation that is used as a substrate, for example plastics such as polyimides.

An initial step in the methods of the invention typically includes the formation of a base layer of the sensor. The base layer can be disposed on the substrate by any desired means, for example by controlled spin coating. In addition, an adhesive may be used if there is not sufficient adhesion between the substrate layer and the base layer. A base layer of insulative material is formed on the substrate, typically by applying the base layer material onto the substrate in liquid form and thereafter spinning the substrate to yield the base layer of thin, substantially uniform thickness. These steps are repeated to build
up the base layer of sufficient thickness, followed by a sequence of photolithographic and/or chemical mask and etch steps to form the conductors discussed below. In an illustrative form, the base layer comprises a thin film sheet of insulative material, such as ceramic or polyimide substrate. The base layer can comprise an alumina substrate, a polyimide substrate, a glass sheet, controlled pore glass, or a planarized plastic liquid crystal polymer. The base layer may be derived from any material containing one or more of a variety of elements including, but not limited to, carbon, nitrogen, oxygen, silicon, sapphire, diamond, aluminum, copper, gallium, arsenic, lanthanum, neodymium, strontium, titanium, yttrium, or combinations thereof. Additionally, the substrate may be coated onto a solid support by a variety of methods well-known in the art including chemical vapor deposition, physical vapor deposition, or spin-coating with materials such as spin glasses, chalcogenides, graphite, silicon dioxide, organic synthetic polymers.

The methods of the invention further include the generation of a conductive layer having one or more sensing elements. Typically these sensing elements are electrodes that are formed by one of the variety of methods known in the art such as photoresist, etching and rinsing to dehne the geometry of the active electrodes. The electrodes can then be made electrochemically active, for example by electrodeposition of Pt black for the working and counter electrode, and silver followed by silver chloride on the reference electrode. A sensor layer such as a sensor chemistry enzyme layer can then be disposed on the sensing layer by electrochemical deposition or a method other than electrochemical deposition such a spin coating, followed by vapor crosslinking, for example with a dialdehyde (glutaraldehyde) or a carbodi-imide.

Electrodes of the invention can be formed from a wide variety of materials known in the art. For example, the electrode may be made of a noble late transition metals. Metals such as gold, platinum, silver, rhodium, iridium, ruthenium, palladium, or osmium can be suitable in various embodiments of the invention. Other compositions such as carbon or mercury can also be useful in certain sensor embodiments. Of these metals, silver, gold, or platinum is typically used as a reference electrode metal. A silver electrode which is subsequently chloridized is typically used as the reference electrode. These metals can be deposited by any means known in the art, including the plasma deposition method cited, supra, or by an electroless method which may involve the deposition of a metal onto a previously metallized region when the substrate is dipped into a solution containing a metal salt and a reducing agent. The electroless method proceeds as the reducing agent donates electrons to the conductive (metallized) surface with the concomitant reduction of the metal salt at the conductive surface. The result is a layer of adsorbed metal. (For additional discussions on electroless methods, see: Wise, E. M. Palladium: Recovery, Properties, and Uses, Academic Press, New York, New York (1988); Wong, K. et al. Plating and Surface Finishing 1988, 75, 70-76; Matsuoka, M. et al. Ibid. 1988, 75, 102-106; and Pearlstein, F. "Electroless Plating," Modern Electroplating, Lowenheim, F. A., Ed., Wiley, New York, N.Y. (1974), Chapter 31.). Such a metal deposition process must yield a structure with good metal to metal adhesion and minimal surface contamination, however, to provide a catalytic metal electrode surface with a high density of active sites. Such a high density of active sites is a property necessary for the efficient redox conversion of an electroactive species such as hydrogen peroxide.

In an exemplary embodiment of the invention, the base layer is initially coated with a thin film conductive layer by electrode deposition, surface sputtering, or other suitable process step. In one embodiment this conductive layer may be provided as a plurality of thin film conductive layers, such as an initial chrome-based layer suitable for chemical adhesion to a polyimide base layer followed by subsequent formation of thin film gold-based and chrome-based layers in sequence. In alternative embodiments, other electrode layer conformations or materials can be used. The conductive layer is then covered, in accordance with conventional photolithographic techniques, with a selected photoresist coating, and a contact mask can be applied over the photoresist coating for suitable photoimaging. The contact mask typically includes one or more conductor trace patterns for appropriate exposure of the photoresist coating, followed by an etch step resulting in a plurality of conductive sensor traces remaining on the base layer. In an illustrative sensor construction designed for use as a subcutaneous glucose sensor, each sensor trace can include three parallel sensor elements corresponding with three separate electrodes such as a working electrode, a counter electrode and a reference electrode.

Portions of the conductive sensor layers are typically covered by an insulative cover layer, typically of a material such as a silicon polymer and/or a polyimide. The insulative cover layer can be applied in any desired manner. In an exemplary procedure, the insulative cover layer is applied in a liquid layer over the sensor traces, after which the substrate is spun to distribute the liquid material as a thin film overlying the sensor traces and extending beyond the marginal edges of the sensor traces in sealed contact with the base layer. This liquid material can then be subjected to one or more suitable radiation and/or chemical and/or heat curing steps as are known in the art. In alternative embodiments, the liquid material can be applied using spray techniques or any other desired means of application. Various insulative layer materials may be used such as photoimagable epoxyacrylate, with an illustrative material comprising a photoimagable polyimide available from OCG, Inc. of West Paterson, N.J., under the product number 7020.

Subsequent to treatment of the sensor elements, one or more additional functional coatings or cover layers can then be applied by any one of a wide variety of methods known in the art, such as spraying, dipping, etc. Some embodiments of the present invention include an analyte modulating layer deposited over the enzyme-containing layer. In addition to its use in modulating the amount of analyte(s) that contacts the active sensor surface, by utilizing an analyte limiting membrane layer, the problem of sensor fouling by extraneous materials is also obviated. As is known in the art, the thickness of the analyte modulating membrane layer can influence the amount of analyte that reaches the active enzyme. Consequently, its application is typically carried out under defined processing conditions, and its dimensional thickness is closely controlled. Microfabrication of the underlying layers can be a factor which affects dimensional control over the analyte modulating membrane layer as well as exact the composition of the analyte limiting membrane layer material itself. In this regard, it has been discovered that several types of copolymers, for example, a copolymer of a siloxane and a nonsiloxane moiety, are particularly useful. These materials can be microdispensed or spin-coated to a controlled thickness. Their final architecture may also be designed by patterning and photolithographic techniques in conformity with the other discrete structures described herein. Examples of these nonsiloxane-siloxane copolymers include, but are not limited to, dimethylsiloxane-alkene oxide, tetramethyldisiloxane-divinylbenzene, tetramethyldisiloxane-ethylene, dimethylsiloxane-silphenylene, dimethylsiloxane-silphenylene oxide, dimethylsiloxane-a-methylstyrene, dimethylsiloxane-bisphenol A carbonate copolymers, or suitable combinations thereof. The percent by weight of the nonsiloxane component of the copolymer can be preselected to any useful value but typically this proportion lies in the range of 40-80 wt %. Among the copolymers listed above, the dimethylsiloxane-bisphenol A carbonate copolymer which comprises 50-55 wt % of the nonsiloxane component is typical. These materials may be purchased from Petrarch Systems, Bristol, Pa. (USA) and are described in this company's products catalog. Other materials which may serve as analyte limiting membrane layers include, but are not limited to, polyurethanes, cellulose acetate, cellulose nitrate, silicone rubber, or combinations of these materials including the siloxane nonsiloxane copolymer, where compatible.

In some embodiments of the invention, the sensor is made by methods which apply an analyte modulating layer that comprises a hydrophilic membrane coating which can regulate the amount of analyte that can contact the enzyme of the sensor layer. For example, the cover layer that is added to the glucose sensors of the invention can comprise a glucose limiting membrane, which regulates the amount of glucose that contacts glucose oxidase enzyme layer on an electrode. Such glucose limiting membranes can be made from a wide variety of materials known to be suitable for such purposes, e.g., silicones such as polydimethyl siloxane, polyurethanes, cellulose acetates, Nation, polyester sulfonic acids (e.g. Kodak AQ), hydrogels or any other membrane known to those skilled in the art that is suitable for such purposes. In certain embodiments of the invention, the analyte modulating layer comprises a linear polyurethane/polyurea polymer stabilized with a branched acrylate copolymer having a central chain and a plurality of side chains coupled to the central chain, wherein at least one side chain comprises a silicone moiety. In some embodiments of the invention pertaining to sensors having hydrogen peroxide recycling capabilities, the membrane layer that is disposed on the glucose oxidase enzyme layer functions to inhibit the release of hydrogen peroxide into the environment in which the sensor is placed and to facilitate the contact between the hydrogen peroxide molecules and the electrode sensing elements.

In some embodiments of the methods of invention, an adhesion promoter layer is disposed between a cover layer (e.g. an analyte modulating membrane layer) and a sensor chemistry layer in order to facilitate their contact and is selected for its ability to increase the stability of the sensor apparatus. As noted herein, compositions of the adhesion promoter layer are selected to provide a number of desirable characteristics in addition to an ability to provide sensor stability. For example, some compositions for use in the adhesion promoter layer are selected to play a role in interference rejection as well as to control mass transfer of the desired analyte. The adhesion promoter layer can be made from any one of a wide variety of materials known in the art to facilitate the bonding between such layers and can be applied by any one of a wide variety of methods known in the art. Typically, the adhesion promoter layer comprises a silane compound such as γ-aminopropyltrimethoxysilane. In certain embodiments of the invention, the adhesion promoting layer and/or the analyte modulating layer comprises an agent selected for its ability to crosslink a siloxane moiety present in a proximal. In other embodiments of the invention, the adhesion promoting layer and/or the analyte modulating layer comprises an agent selected for its ability to crosslink an amine or carboxyl moiety of a protein present in a proximal layer. In an optional embodiment, the AP layer further comprises Polydimethyl Siloxane (PDMS), a polymer typically present in analyte modulating layers such as a glucose limiting membrane. In illustrative embodiments the formulation comprises 0.5-20% PDMS, typically 5-15% PDMS, and most typically 10% PDMS. The addition of PDMS to the AP layer can be advantageous in contexts where it diminishes the possibility of holes or gaps occurring in the AP layer as the sensor is manufactured.

As noted above, a coupling reagent commonly used for promoting adhesion between sensor layers is γ-aminopropyltrimethoxysilane. The silane compound is usually mixed with a suitable solvent to form a liquid mixture. The liquid mixture can then be applied or established on the wafer or planar sensing device by any number of ways including, but not limited to, spin-coating, dip-coating, spray-coating, and microdispensing. The microdispensing process can be carried out as an automated process in which microspots of material are dispensed at multiple preselected areas of the device. In addition, photolithographic techniques such as "lift-off or using a photoresist cap may be used to localize and define the geometry of the resulting permselective film (i.e. a film having a selective permeability). Solvents suitable for use in forming the silane mixtures include aqueous as well as water-miscible organic solvents, and mixtures thereof. Alcoholic water-miscible organic solvents and aqueous mixtures thereof are particularly useful. These solvent mixtures may further comprise nonionic surfactants, such as polyethylene glycols (PEG) having a for example a molecular weight in the range of 200 to 6,000. The addition of these surfactants to the liquid mixtures, at a concentration of 0.005 to 0.2 g/dL of the mixture, aids in planarizing the resulting thin films. Also, plasma treatment of the wafer surface prior to the application of the silane reagent can provide a modified surface which promotes a more planar established layer. Water-immiscible organic solvents may also be used in preparing solutions of the silane compound. Examples of these organic solvents include, but are not limited to, diphenylether, benzene, toluene, methylene chloride, dichloroethane, trichloroethane, tetrachloroethane, chlorobenzene, dichlorobenzene, or mixtures thereof. When protic solvents or mixtures thereof are used, the water eventually causes hydrolysis of the alkoxy groups to yield organosilicon hydroxides (especially when n=1) which condense to form poly(organosiloxanes). These hydrolyzed silane reagents are also able to condense with polar groups, such as hydroxyls, which may be present on the substrate surface. When aprotic solvents are used, atmospheric moisture may be sufficient to hydrolyze the alkoxy groups present initially on the silane reagent. The R' group of the silane compound (where n=1 or 2) is chosen to be functionally compatible with the additional layers which are subsequently applied. The R' group usually contains a terminal amine group useful for the covalent attachment of an enzyme to the substrate surface (a compound, such as glutaraldehyde, for example, may be used as a linking agent as described by Murakami, T. et al., Analytical Letters 1986, 19, 1973-86).

Various publication citations are referenced throughout the specification. In addition, certain text from related art is reproduced herein to more clearly delineate the various embodiments of the invention.

### EXAMPLES

The following examples are given to aid in understanding the invention, but it is to be understood that the invention is not limited to the particular materials or procedures of examples. All materials used in the examples were obtained from commercial sources.

### EXAMPLE 1: SYNTHESIS AND CHARACTERIZATION OF ILLUSTRATIVE LINEAR POLYUREA/POLYURETHANE POLYMERS:

The disclosure provided herein in combination with what is known in that art confirms functional linear polyurethane/polyurea polymers can be made from a number of formulations, for example those disclosed in U.S. Patent Nos. 5,777,060; 5,882,494; 6,642,015; and PCT publications WO 96/30431; WO 96/18115; WO 98/13685; and WO 98/17995. Certain of these polymers provide formulations useful as a glucose limiting membrane (GLM).

### Standard GLM formulations comprise, for example:

25 mol% polymethylhydrosiloxane (PDMS), trimethylsilyl terminated, 25-35 [centistokes] mm²/s;
25 mol% polypropylene glycol diamine (Jeffamine 600, a polyoxyalkyleneamine with an approximate molecular weight of 600); and
50 mol% of a diisocyanate (e.g., 4,4'-diisocyanate).

However, polymers formed from such reagents have been found to have less than ideal properties with regard to thermal degradation and/ or oxidation. For example, as shown in the data provided in FIG. 3, films of this polymer stored for a month at 45°C and 60°C show large decreases in molecular weight (25% to 69%, respectively).

As discussed herein, by using a "standard GLM" noted above as a starting point, we have generated a new polymeric formulation that improves the oxidative and/or thermal stability of the polymeric compositions that form the analyte modulating layers that are one of the key components of in glucose sensor embodiments. By improving the oxidative and/or thermal stability of the polymeric compositions, the new formulations provide a more robust glucose sensor. The synthesis of the glucose limiting polymers that form the glucose limiting membrane is summarized below.

### TYPICAL ILLUSTRATIVE PROCEDURE

The following describes the synthesis procedure of Stabilized Glucose Limiting Polymer used for coating glucose sensors.

### Typical Materials

Tetrahydrofuran (THF) Inhibitor free, low moisture.
Poly (propylene glycol-B-ethylene glycol-B-propylene glycol) bis (2 aminopropyl ether) (Average Molecular Weight ∼600) (CAS # 6560536-9) (Aldrich or Huntsman (listed as Jeffamine ED), dried.
Polydimethylsiloxane, aminopropyldimethyl terminated (Estimated Molecular Weight ∼ 2200 to 4000 g/ml) (CAS # 106214-84-0) dried.
Dibutyltin bis (2-ethylhexanoate)
4,4'-Methylenebis (cyclohexyl isocyanate) (CAS # 5124-30-1)
Methylenebis(2,6-di-tert-butylphenol) (CAS#118-82-1) (8081284)
Distilled or Deionized Water and Nitrogen gas.

Typical chemical synthetic lab equipment includes a jacketed resin kettle/flask with inlet/outlet adapters, mechanical stirrer, syringe pump. Water circulating temperature controller, teflon luer lock flexible cannula, disposable polypropylene syringes (20 ml/50 ml, 50 ml, 50ml/100ml/150ml ground glass-luer lock syringes, stainless steel syringe needles 16g, 12 inch), 24/40 rubber septa, gas inlet adapter/tube, ground glass stirring rod & paddle, nitrogen gas, 4 liter beakers (2), magnetic stir bars, magnetic stirrer/hotplate, 4L industrial blender, wire screen.

### Synthesis of Polymer

The following synthesis procedures describe the formulation of 360 grams of Stabilized Glucose Limiting Polymer. This reaction can be scaled up to 600 grams and scaled down to 60 grams accordingly.

### Set up of polymer synthesis:

Hot air or oven-dry the following:
a. 3.5-Liter jacketed resin kettle with 4-necked, 24/40 reaction head
b. Rubber O-ring
c. Stirring rod and stirrer bearing adapter for mechanical stirring apparatus

Dry materials. Store dried materials in dessicator until use.

Carefully connect reaction apparatus as follows:
Insert stirring rod with paddle through bearing and connect bearing to the center joint of the reaction kettle cover. Place reaction flask head onto the jacketed flask with the O-ring in place. Seal the remaining openings with 24/40 rubber septa. A needle-type Nitrogen line or tubing can be attached to the rubber septum or gas inlet tube at the top of the condenser. Initiate the flow of dry nitrogen and if necessary dry the system in place with the aid of a forced hot air dryer.

Into a preweighed 50 ml polypropylene syringe measure 122.76 ± 0.15 grams (∼120 ml) of poly (propylene glycol-β-ethylene glycol-β-propylene glycol), bis (2-aminopropyl terminated) (MW∼600) (Jeffamine 600) (204.6 mmol, 0.75 eq). The Jeffamine should be withdrawn with a syringe needle through the double septa of the sealed flask (keep a positive pressure of nitrogen on the Jeffamine flask to avoid contact with air/moisture). Add this to the reaction flask through the rubber septum.

Into a preweighed polypropylene syringe measure 170.25 ± 0.15 grams (∼156 ml) polydimethylsiloxane, aminopropyl dimethyl terminated (68.1 mmoI, 0.25 eq). The siloxane should be withdrawn with a syringe needle through the double septa of the sealed flask (keep a positive pressure of nitrogen on the siloxane flask to avoid contact with air/moisture). Add this to the reaction flask through the rubber septum.

Weigh 729 ± 15 mg dibutyltin-bis- (2-ethyl hexanoate), onto a tared disposable weighing dish or paper and transfer to the reaction vessel through one of the openings in the reaction head and reseal the vessel.

Gently begin warming the reaction vessel to 40 ± 5°C, with the aid of the recirculating water bath and carefully transfer at least 600 ml distilled or low moisture bottled THF (bottled THF must come from freshly opened bottle; draw into a syringe or cannulate through septum of bottle) into the reaction vessel using a syringe with needle, minimizing exposure to air. Turn on stirrer to begin mixing. Additional THF (up to 2000ml total) may be added during the course of the reaction to facilitate mixture stirring. Note actual volume of THF used in traveler. Allow the reaction solution to equilibrate for 30 ± 5 minutes.

Weigh 729 ± 15 mg dibutyltin-bis- (2-ethyl hexanoate), onto a tared disposable weighing dish or paper and transfer to the reaction vessel through one of the openings in the reaction head and reseal the vessel.

Into a preweighed syringe measure 72.25 ± 0.15 grams of 4,4'-methylenebis (cyclohexyl isocyanate) (∼66 ML) (270 mmol, 1 .01 eq). The cyclohexyl isocyanate should be withdrawn with a syringe needle through the septum of the bottle (keep a positive pressure of nitrogen on the cyclohexyl isocyanate flask to avoid contact with moisture). Place the syringe into the syringe pump holding device and affix the luer locking Teflon cannula to the syringe. Place the flexible cannula through the rubber septum and lower the delivery end near-to the reaction medium. Set the delivery rate so that the isocyanate is added at a steady rate over the course of 25 minutes (+/-3 minutes).

Upon completion of the addition (∼25 minutes), the syringe is flushed with 15-45 ml dry THF and added to the reaction. The circulating water bath temperature is increased to 60 ± 5°C from 40°C and the reaction is allowed to proceed for an additional 12-18 hours.

Weigh 1.8 grams Methylenebis(2,6-di-tert-butylphenol) (CAS#118-82-1) in a small vial. Add 10mL fresh THF to dissolve, and then add to the reaction and maintain stirring & heating for an additional 8 hours.

Add 96 ± 15 ml of de-ionized water to the reaction and maintain stirring & heating for an additional 12-15 hours.

### Typical Work up and isolation of polymer

The temperature bath is shut off and the circulating water lines disconnected. Stirring is continued for at least 15 minutes allowing the solution to cool. Separately, a 4-liter industrial blender is filled with 3 liters of deionized or distilled water. One half of the reaction mixture is added to the blender and the blender is covered and set on low (∼15,000rpm) for 15 seconds, then set on medium (∼18,000rpm) for 30 seconds. The mixture is poured through a wire screen and the water discarded. The polymer precipitate is placed back into the blender and 3 liters of clean deionized or distilled water is added.

The blender is set on medium for 30 seconds and the mixture is then filtered through a wire screen and the water discarded. Repeat this procedure for the remainder of the reaction mixture.

Two 4 liter beakers are filled to approximately the 3.0-liter mark with distilled or deionized water. The polymer is divided into two portions and each portion is added to a 4-liter beaker. The beakers are placed onto hot plate-stirrers and a magnetic stir bar added to each. The mixtures are stirred and heated to a gentle boil and maintained for 60-120 minutes. The beakers are removed, and the polymer separated by pouring through a fine-mesh screen while hot. The reaction vessel is placed onto a cork ring and filled with water. The water bath is re-connected and the flask heated to 60°C ± 5° C. for at least one hour to loosen the polymer residuals from the glass.

The polymer is patted dry and placed into a large crystallization dish, placed into a vacuum oven and heated (60 ± 2°C) under vacuum (25-30 "Hg) for 12-18 hours.

The dried polymer is weighed, the weight is recorded and the polymer placed into a properly labeled container.

A 6-7 gram sample is placed in a properly labeled container. This sample can then be submitted to, for example an Analytical Chemistry Lab for testing.

### PROCEDURES USED IN EMBODIMENTS SHOWN IN FIG. 3

The following describes the synthesis procedure of Stabilized Glucose Limiting Polymers used in the data shown in FIG. 3.
1. A reaction flask is charged with 122.76 grams JeffamineED (CAS#6560536-9), 170.25 grams aminopropyldimethyl-terminated Polydimethylsiloxane (CAS# 106214-84-0), 0.729 grams Dibutyltinbis(2-ethylhexanoate) (CAS# 2781-10-4), and 600 to 2000 milliliters of Tetrahydrofuran (CAS# 109-99-9). The flask is put under nitrogen gas and heated to 40C with stirring.
2. 72.25 grams of 4,4-Methylenebis(cyclohexyl isocyanate) (CAS# 5124-30-1) is added dropwise to the mixture over 25 minutes. The mixture is heated to 60C and stirred an additional 12-18 hours.
3.The antioxidant compound is then dissolved in tetrahydrofuran and added to the mixture in one portion. For example, 1.80 grams of 4,4-methylenebis(2,6-di-tert-butylphenol) (CAS# 118-82-1) is dissolved in 10 milliliters of tetrahydrofuran and added to the mixture in one portion. This mixture is stirred at 60C for an additional 8 hours.
4. 100mL of deionized or distilled water is then added in one portion to the mixture and the mixture is stirred an additional 12-15 hours at 60C.
5. The mixture is cooled at least 15 minutes and then ½ the volume is precipitated into 3 liters of water in an industrial blender. The polymer is collected, and blended again with a fresh 3 liters of water. This step is repeated for the remainder of the mixture. Both portions of polymer are collected and then dried overnight in a vacuum oven (25mmHg) at 70C.
6. As shown in FIGS. 3A-3E, the addition of small amounts of several anti-oxidant compounds during the reaction synthesis greatly improves the thermal stability of GLP. These materials are readily available commercially and illustrative embodiments include AO1(4,4-Methylenebis(2,6-di-tert-butylphenol) [CAS# 118-82-1], AO2(2,2-Ethylidenebis(4,6-di-tert-butylphenol)[CAS#35958-30-6], and AO3(2,2-Methylenebis(6-tert-butyl-4-methylphenol)[CAS#119-47-1]. These materials are added to the initial reactant mixture of the polymer synthesis. They all have functional groups that may react to some degree with the existing polymer reactants. They are typically added in small amounts (∼0.5% by weight) and do not adversely affect the properties of the polymer product.

The improved thermal stability may be seen in graphs 1 and 2 in FIGS. 3A and 3B respectively. Graph 1 as shown in FIG. 3A shows that the current GLP drops in molecular weight by 25% (197kD to 148kD) after 4 weeks of storage at 45C. The 3 antioxidant-reacted GLP's show much less molecular weight decrease (<5%) over the same period. AO1-reacted GLP drops only 1% (125kD to 124kD), AO2-reacted GLP drops 4% (119kD to 114kD), and AO3-reacted GLP shows no detectable change in molecular weight (139kD to 150kD).

Graph 2 as shown in FIG. 3B shows how the current GLP molecular weight drops dramatically(69%) after one month of storage at 60C, while the 3 antioxidant reacted GLP's again show much improved stability. AO1-reacted GLP drops only 3% (125kD to 121kD), AO2-reacted GLP drops 24% (199kD to 90kD), and AO3 drops 9% (139kD to 126kD).

We speculated that the higher molecular weight of the current GLP (197kD) might account for its decreased stability relative to the lower molecular weight antioxidant reacted polymers(119kD to 139kD). Therefore we made 3 AO2-reacted polymers of different molecular weight: low Mw (135kD), medium Mw (172kD), and high Mw(324kD). Graph 3 as shown in FIG. 3C shows the thermal stability of these polymers at 60C and suggests that the antioxidant is in fact responsible for most of the stabilizing effect, rather than the lower initial molecular weight. The very high molecular weight antioxidant-reacted GLP (324kD) drops only 42% after one month, versus 69% for the current GLP (initial Mw = 197kD). The low Mw (135kD) AO2-reacted GLP shows only a 24% decrease (135kD to 103kD) that is similar to the decrease (28%) of the medium Mw (172kD to 124kD) GLP. Again, both show a great improvement over the current GLP decrease of 69%.

FIGS 4A-and 4B provide graphs showing the results of an accelerated aging study in which groups of sensors were heated at 45 degrees centigrade for 4.7 months. FIG. 4A shows studies from a group of glucose sensors formed using a conventional GLM composition to which no polyurethane/polyurea polymer stabilizing compound was added. FIG. 4B shows studies from a group of sensors formed using a GLM composition to which a polyurethane/polyurea polymer stabilizing compound has been added (in this embodiment, the compound is covalently bound to the polymers in this composition). Effects of the stabilizing compound can be observed, for example, by comparing the range of individual sensor Isig values in the sensors shown in FIG. 4A ("*") as compared the range of individual sensor Isig values in the sensors shown in FIG. 4B ("**").
Figure 5A provides diagrams of compounds useful to make typical polymer composition embodiments of the invention. Figure 5B shows the polymer structures generated by mixing these compounds. Polydimethylsiloxane (PDMS) belongs to a group of polymeric organosilicon compounds that are commonly referred to as silicones. PDMS is a widely used silicon-based organic polymer, and is particularly known for its unusual rheological (or flow) properties. JEFFAMINE® polyoxyalkyleneamines are a part of a family of polyether compound products. They contain primary amino groups attached to the terminus of a polyether backbone. They are thus "polyether amines." The polyether backbone is based either on propylene oxide (PO), ethylene oxide (EO), or mixed EO/PO. The JEFFAMINE® family consists of monoamines, diamines, and triamines, which are available in a variety of molecular weights, ranging up to 5,000. Some Jeffamines may contain other backbone segments and varied reactivity provided by hindering the primary amine or through secondary amine functionality. Hexamethylene diisocyanate (HDI) is an organic compound in the class of isocyanates, more specifically an aliphatic diisocyanate. Mixtures of the compounds shown in FIG. 5 can be combined with polymer stabilizing compounds that include those comprising the structures disclosed in FIG. 6A. FIG. 6B shows the structure of typical Glucose Limiting Polymer Embodiment comprising a stabilizing anti-oxidant agent.

The following Table 1 shows the results of a thermal Stability Study, one where the Glucose Limiting Polymer was physically mixed (not covalently bonded) with 0.5 % (weight/weight) Pyrogallol.

**TABLE 1**

| | Storage at 60C (Mw in kD) | | | | |
|---|---|---|---|---|---|
| Time = | 0 | 7 days | 2wks | 3 wks | 4wks |
| sample | | | | | |
| | | | | | |
| GLM mixed w/pyrogallol(0.5%) | 222 | 215 | 198 | 199 | 193 |

Pyrogallol is a strong oxidizer. Without being bound by any theory or mechanism of action, pyrogallol may stabilize the GLP/GLM through a different approach (i.e. pyrogallol reacts with all the environmental oxygen, so that there is no more oxygen to attack the GLM).

Those of skill in this art understand that the non-limiting examples provided herein are illustrative and that a variety of embodiments of the invention can be made using conventional process variations. For example, another formulation that can be used in embodiments of the invention is termed a "half permeable GLM", due to the observation that its glucose permeability is one-half of the standard formulation immediately above. In the standard GLM, the Jeffamine/PDMS ration = 3/1 (mole ratio). In contrast, in the "half permeable GLM", this ratio is altered so that the Jeffamine/PDMS = 12/1. This half-permeable GLM is can be used for example to reduce the weight % of GLM-urea in an overall polymer blend in order to reach a particular Isig (or glucose permeability). In addition, the molecular weights of the final polymers can be modulated by modulating the reaction conditions according to art accepted practices. For example the variable molecular weight polymers that are used in the data shown in Fig. 3C are all made from the exact same formulation and reaction conditions, except that the solvent (Tetrahydrofuran) amount was varied for each synthesis. The low Mw polymer used 400mL THF, the high Mw polymer used 200mL THF, and the mid Mw polymer used 340mL THF. The "control-current GLP" is considered a mid-range Mw polymer.

In addition, the stabilizing agents disclosed herein can be both covalently bound to the polymer compositions, or alternatively, entrapped within the polymer compositions. Embodiments where the stabilizing agent is covalently bound to the polymer compositions can be formed by including the stabilizing agent in the polymerization reaction mixture. Embodiments where the stabilizing agent is entrapped within the polymer compositions can be formed by simply mixing (i.e. physically mixing, with no chemical reaction) the agent into the already formed polymer (e.g. a GLM that has been pre-synthesized/precipitated/dried using a standard process). Depending upon the stabilizing agent selected, one may prefer physically mixed preparation over a covalently bound preparation (or vice versa). For example, a pyrogallol stabilized polymer is probably best prepared as a physically mixed preparation, because this agent can crosslink the polymer if added during polymer synthesis.

### EXAMPLE 2: ACCELERATED AGING PROTOCOLS

Accelerated aging protocols comprise assays that use aggravated conditions of heat, oxygen, sunlight, vibration, etc. to speed up the normal aging processes of items. Such assays are typically used to help determine the long term effects of expected levels of a stress (e.g. exposure to heat, oxidating agents, radiation etc.) within a shorter time, usually in a laboratory by controlled standard test methods. Such assays therefore estimate the useful lifespan of a product or its shelf life when actual lifespan data is unavailable. Physical testing or chemical testing is carried out by subjecting the product to 1)representative levels of stress for long time periods, 2) unusually high levels of stress used to accelerate the effects of natural aging, or 3) levels of stress that intentionally force failures (for further analysis). For example, in such assays, polymers are often kept at elevated temperatures, in order to accelerate chemical breakdown.

A variety of accelerated aging protocols can be used to examine different embodiments of the invention. Typically, such assays are designed to mimic the environment to which the polymer is exposed (e.g. the shipping and storage of a sensor that comprises the polymer). In one illustrative example of such an assay, the total aging time is 140.2 days, which includes 60°C for 6 hrs (shipping); 50°C for 10 days (shipping); and 45°C for 129.95 days (storage).

## Claims

1. Use of a composition of matter in an analyte sensor device comprising a polymer formed from a mixture comprising:
a diisocyanate;
a siloxane;
a hydrophilic diol or hydrophilic diamine; and
a polymer stabilizing compound, wherein the polymer stabilizing compound:
has a molecular weight of less than 1000 g/mol;
comprises a benzyl ring having at least one hydroxyl moiety (ArOH), **characterized in that**
the amount of said hydrophilic diol or diamine is 10% to 80% (mol%) relative to the diisocyanate.

2. The use of a composition according to claim 1, wherein the polymer is formed from a mixture comprising:
45-55 mol% diisocyanate;
10-20 mol% siloxane;
30-45 mol% hydrophilic diol or hydrophilic diamine; and
0.1-5 weight % polymer stabilizing compound.

3. The use of a composition according to claim 1, wherein the polymer stabilizing compound comprises at least two benzyl rings having at least one hydroxyl moiety.

4. The use of a composition according to claim 1, wherein the polymer stabilizing compound comprises:
pyrogallol;
catechol;
2,2'-Methylenebis(6-tert-butyl-4-methylphenol;
2,2'-Ethylene-bis(4,6-di-tert-butylphenol); or
4,4'-Methylenebis (2,6-di-tert-butylphenol).

5. The use of a composition according to claim 1, wherein the polymer stabilizing compound is
a) covalently coupled at one or both ends of the polymer or
b) not covalently coupled to the polyurethane/ polyurea polymer; and entrapped within a plurality of polyurethane/polyurea polymers.

6. The use of a composition according to claim 1, wherein the polymer is further mixed with a branched acrylate polymer; at a ratio of between 1:1 and 1:20 by weight %.

7. An analyte sensor system comprising:
a probe adapted to be inserted in vivo, wherein the probe includes an electrode array comprising:
a working electrode, a counter electrode; and a reference electrode;
an analyte sensing layer disposed on the working electrode;
an analyte modulating layer disposed on the analyte sensing layer, wherein the analyte modulating layer comprises a polyurethane/ polyurea polymer formed from a mixture comprising:
(a) a diisocyanate;
(b) a hydrophilic polymer comprising a hydrophilic diol or hydrophilic diamine;
(c) a siloxane having an amino, hydroxyl or carboxylic acid functional group at a terminus; and
(d) a polyurethane/polyurea polymer stabilizing compound selected for its ability to inhibit thermal and oxidative degradation of polyurethane/ polyurea polymers formed from the mixture, wherein the polyurethane/ polyurea polymer stabilizing compound:
has a molecular weight of less than 1000 g/mol;
comprises a benzyl ring having at least one hydroxyl moiety (ArOH).

8. The analyte sensor system of claim 7, wherein the polyurethane/polyurea polymer stabilizing compound is
a) covalently coupled to the polyurethane/polyurea polymer or
b) not covalently coupled to the polyurethane/polyurea polymer; and entrapped within a plurality of polyurethane/polyurea polymers.

9. The analyte sensor system of claim 7, wherein the polyurethane/polyurea polymer stabilizing compound exhibits an antioxidant activity.

10. The analyte sensor system of claim 7, wherein the polyurethane/polyurea polymer stabilizing compound
a) comprises at least two benzyl rings having at least one hydroxyl moiety or
b) is a compound as defined in claim 4.

11. The analyte sensor system of claim 7, wherein the polyurethane/polyurea polymer comprises:
45-55 mol% diisocyanate;
10-20 mol% siloxane;
30-45 mol% hydrophilic diol or hydrophilic diamine; and
0.1-5 weight % polyurethane/polyurea polymer stabilizing compound.

12. The analyte sensor system of claim 7, wherein the polyurethane/polyurea polymer stabilizing compound comprises a benzyl ring having at least two hydroxyl moieties.

13. The analyte sensor system of claim 7, further comprising at least one of:
an interference rejection membrane;
a protein layer;
an adhesion promoting layer disposed on the analyte sensing layer, wherein the adhesion promoting layer promotes the adhesion between the analyte sensing layer and the analyte modulating layer; or
a cover layer wherein the cover layer comprises an aperture positioned on the cover layer so as to facilitate an analyte present in the mammal contacting and diffusing through an analyte modulating layer; and contacting the analyte sensing layer.

14. The analyte sensor system of claim 7, further comprising a probe platform and wherein the first probe comprises:
a first electrode array comprising a working electrode, a counter electrode and a reference electrode; and
a second electrode array comprising a working electrode, a counter electrode and a reference electrode;
a second probe coupled to the probe platform and adapted to be inserted in vivo, wherein the second probe comprises:
a third electrode array comprising a working electrode, a counter electrode and a reference electrode; and
a fourth electrode array comprising a working electrode, a counter electrode and a reference electrode;
wherein the first, second, third and fourth electrode arrays are configured to be electronically independent of one another.

15. The use of a composition of matter comprising a polymer formed from a mixture comprising:
a diisocyanate;
a siloxane;
a hydrophilic diol or hydrophilic diamine; and
a polymer stabilizing compound, wherein the polymer stabilizing compound:
has a molecular weight of less than 1000 g/mol;
comprises a benzyl ring having at least one hydroxyl moiety (ArOH) for the manufacturing of a device for the detection of an analyte.

## Patentansprüche

1. Verwendung einer Materialzusammensetzung in einer Analytsensorvorrichtung, die ein Polymer umfasst, das aus einer Mischung gebildet ist, die Folgendes umfasst:
ein Diisocyanat;
ein Siloxan;
ein hydrophiles Diol oder hydrophiles Diamin; und
eine polymerstabilisierende Verbindung, wobei die polymerstabilisierende Verbindung:
ein Molekulargewicht von weniger als 1000 g/mol aufweist;
einen Benzylring mit mindestens einer Hydroxyleinheit (ArOH) umfasst,
**dadurch gekennzeichnet, dass**
die Menge des hydrophilen Diols oder Diamins relativ zu dem Diisocyanat 10 % bis 80 % (Mol-%) beträgt.

2. Verwendung einer Zusammensetzung nach Anspruch 1, wobei das Polymer aus einer Mischung gebildet ist, die Folgendes umfasst:
45-55 Mol-% Diisocyanat;
10-20 Mol-% Siloxan;
30-45 Mol-% hydrophiles Diol oder hydrophiles Diamin; und
0,1-5 Gew.-% polymerstabilisierende Verbindung.

3. Verwendung einer Zusammensetzung nach Anspruch 1, wobei die polymerstabilisierende Verbindung mindestens zwei Benzylringe mit mindestens einer Hydroxyleinheit umfasst.

4. Verwendung einer Zusammensetzung nach Anspruch 1, wobei die polymerstabilisierende Verbindung Folgendes umfasst:
Pyrogallol;
Catechol;
2,2'-Methylenbis(6-tert-butyl-4-methylphenol;
2,2'-Ethylen-bis(4,6-di-tert-butylphenol); oder
4,4'-Methylenbis(2,6-di-tert-butylphenol).

5. Verwendung einer Zusammensetzung nach Anspruch 1, wobei die polymerstabilisierende Verbindung
a) kovalent an eines oder beide Enden des Polymers gebunden ist oder
b) nicht kovalent an das Polyurethan/Polyharnstoff-Polymer gebunden; und innerhalb einer Vielzahl von Polyurethan/Polyharnstoff-Polymeren eingeschlossen ist.

6. Verwendung einer Zusammensetzung nach Anspruch 1, wobei das Polymer ferner mit einem verzweigten Acrylatpolymer gemischt ist; in einem Gew.-%-Verhältnis zwischen 1:1 und 1:20.

7. Analytsensorsystem, umfassend:
eine Sonde, die zum Einführen in vivo ausgelegt ist, wobei die Sonde eine Elektrodenanordnung einschließt, die Folgendes umfasst:
eine Arbeitselektrode, eine Gegenelektrode und eine Bezugselektrode;
eine Analyterkennungsschicht, die auf der Arbeitselektrode angeordnet ist;
eine Analytmodulierungsschicht, die auf der Analyterkennungsschicht angeordnet ist, wobei die Analytmodulierungsschicht ein Polyurethan/Polyharnstoff-Polymer umfasst, das aus einer Mischung gebildet ist, die Folgendes umfasst:
(a) ein Diisocyanat;
(b) ein hydrophiles Polymer, umfassend ein hydrophiles Diol oder hydrophiles Diamin;
(c) ein Siloxan mit einer funktionellen Amino-, Hydroxyl- oder Carbonsäuregruppe an einem Ende; und
(d) eine Polyurethan/Polyharnstoff-Polymer stabilisierende Verbindung, ausgewählt aufgrund ihrer Fähigkeit, thermischen und oxidativen Abbau von aus der Mischung gebildeten Polyurethan/Polyharnstoff-Polymeren zu hemmen, wobei die Polyurethan/Polyharnstoff-Polymer stabilisierende Verbindung:
ein Molekulargewicht von weniger als 1000 g/mol aufweist;
einen Benzylring mit mindestens einer Hydroxyleinheit (ArOH) umfasst.

8. Analytsensorsystem nach Anspruch 7, wobei die Polyurethan/Polyharnstoff-Polymer stabilisierende Verbindung
a) kovalent an das Polyurethan/Polyharnstoff-Polymer gebunden ist oder
b) nicht kovalent an das Polyurethan/Polyharnstoff-Polymer gebunden; und innerhalb einer Vielzahl von Polyurethan/Polyharnstoff-Polymeren eingeschlossen ist.

9. Analytsensorsystem nach Anspruch 7, wobei die Polyurethan/Polyharnstoff-Polymer stabilisierende Verbindung eine antioxidative Aktivität vorweist.

10. Analytsensorsystem nach Anspruch 7, wobei die Polyurethan/Polyharnstoff-Polymer stabilisierende Verbindung
a) mindestens zwei Benzylringe mit mindestens einer Hydroxyleinheit umfasst oder
b) eine Verbindung wie in Anspruch 4 definiert ist.

11. Analytsensorsystem nach Anspruch 7, wobei das Polyurethan/Polyharnstoff-Polymer Folgendes umfasst:
45-55 Mol-% Diisocyanat;
10-20 Mol-% Siloxan;
30-45 Mol-% hydrophiles Diol oder hydrophiles Diamin; und
0,1-5 Gew.-% Polyurethan/Polyharnstoff-Polymer stabilisierende Verbindung.

12. Analytsensorsystem nach Anspruch 7, wobei die Polyurethan/Polyharnstoff-Polymer stabilisierende Verbindung einen Benzylring mit mindestens zwei Hydroxyleinheiten umfasst.

13. Analytsensorsystem nach Anspruch 7, das ferner mindestens eines von Folgendem umfasst:
eine Interferenzunterdrückungsmembran;
eine Proteinschicht;
eine Haftungsförderungsschicht, die auf der Analyterkennungsschicht angeordnet ist, wobei die Haftungsförderungsschicht die Haftung zwischen der Analyterkennungsschicht und der Analytmodulierungsschicht fördert; oder
eine Deckschicht, wobei die Deckschicht eine Öffnung umfasst, die auf der Deckschicht angeordnet ist, um zu erleichtern, dass ein in dem Säugetier vorhandener Analyt mit einer Analytmodulierungsschicht in Kontakt kommt und durch sie diffundiert; und mit der Analyterkennungsschicht in Kontakt kommt.

14. Analytsensorsystem nach Anspruch 7, das ferner eine Sondenplattform umfasst und wobei die erste Sonde Folgendes umfasst:
eine erste Elektrodenanordnung, die eine Arbeitselektrode, eine Gegenelektrode und eine Bezugselektrode umfasst; und
eine zweite Elektrodenanordnung, die eine Arbeitselektrode, eine Gegenelektrode und eine Bezugselektrode umfasst;
eine zweite Sonde, die mit der Sondenplattform verbunden und ausgelegt ist, um in vivo eingeführt zu werden, wobei die zweite Sonde Folgendes umfasst:
eine dritte Elektrodenanordnung, die eine Arbeitselektrode, eine Gegenelektrode und eine Bezugselektrode umfasst; und
eine vierte Elektrodenanordnung, die eine Arbeitselektrode, eine Gegenelektrode und eine Bezugselektrode umfasst;
wobei die erste, zweite, dritte und vierte Elektrodenanordnung so konfiguriert sind, dass sie elektronisch unabhängig voneinander sind.

15. Verwendung einer Materialzusammensetzung, die ein Polymer umfasst, das aus einer Mischung gebildet ist, die Folgendes umfasst:
ein Diisocyanat;
ein Siloxan;
ein hydrophiles Diol oder hydrophiles Diamin; und
eine polymerstabilisierende Verbindung, wobei die polymerstabilisierende Verbindung:
ein Molekulargewicht von weniger als 1000 g/mol aufweist;
einen Benzylring mit mindestens einer Hydroxyleinheit (ArOH) für die Fertigung einer Vorrichtung zum Detektieren eines Analyten umfasst.

## Revendications

1. Utilisation d'une composition de matière dans un dispositif capteur d'analyte comprenant un polymère formé à partir d'un mélange comprenant :
un diisocyanate ;
un siloxane ;
un diol hydrophile ou une diamine hydrophile ; et
un composé de stabilisation de polymère, dans laquelle le composé de stabilisation de polymère :
a une masse moléculaire inférieure à 1000 g/mol ;
comprend un cycle benzyle ayant au moins un fragment hydroxyle (ArOH),
**caractérisée en ce que**
la quantité dudit diol hydrophile ou de ladite diamine hydrophile va de 10 % à 80 % (% molaires) par rapport au diisocyanate.

2. Utilisation d'une composition selon la revendication 1, dans laquelle le polymère est formé à partir d'un mélange comprenant :
45 à 55 % molaires de diisocyanate ;
10 à 20 % molaires de siloxane ;
30 à 45 % molaires de diol hydrophile ou de diamine hydrophile ; et
0,1 à 5 % en poids de composé de stabilisation de polymère.

3. Utilisation d'une composition selon la revendication 1, dans laquelle le composé de stabilisation de polymère comprend au moins deux cycles benzyle ayant au moins un fragment hydroxyle.

4. Utilisation d'une composition selon la revendication 1, dans laquelle le composé de stabilisation de polymère comprend :
du pyrogallol ;
du catéchol ;
du 2,2'-méthylènebis(6-tert-butyl-4-méthylphénol) ;
du 2,2'-éthylène-bis(4,6-di-tert-butylphénol) ; ou
du 4,4'-méthylènebis (2,6-di-tert-butylphénol).

5. Utilisation d'une composition selon la revendication 1, dans laquelle le composé de stabilisation de polymère est
a) couplé par covalence à l'une et/ou l'autre des extrémités du polymère ou
b) non couplé par covalence au polymère polyuréthane/polyurée ; et piégé au sein d'une pluralité de polymères polyuréthane/polyurée.

6. Utilisation d'une composition selon la revendication 1, dans laquelle le polymère est en outre mélangé à un polymère acrylate ramifié ; à un rapport compris entre 1:1 et 1:20 % en poids.

7. Système capteur d'analyte comprenant :
une sonde conçue pour être insérée in vivo, dans lequel la sonde inclut un réseau d'électrodes comprenant :
une électrode de travail, une contre-électrode ; et une électrode de référence ;
une couche de détection d'analyte disposée sur l'électrode de travail ;
une couche de modulation d'analyte disposée sur la couche de détection d'analyte, dans lequel la couche de modulation d'analyte comprend un polymère polyuréthane/polyurée formé à partir d'un mélange comprenant :
(a) un diisocyanate ;
(b) un polymère hydrophile comprenant un diol hydrophile ou une diamine hydrophile ;
(c) un siloxane ayant un groupe fonctionnel amino, hydroxyle ou acide carboxylique au niveau d'une terminaison ; et
(d) un composé de stabilisation de polymère polyuréthane/polyurée choisi pour sa capacité à inhiber une dégradation par la chaleur et par oxydation des polymères polyuréthane/polyurée formés à partir du mélange, dans lequel le composé de stabilisation de polymère polyuréthane/polyurée :
a une masse moléculaire inférieure à 1000 g/mol ;
comprend un cycle benzyle ayant au moins un fragment hydroxyle (ArOH).

8. Système capteur d'analyte selon la revendication 7, dans lequel le composé de stabilisation de polymère polyuréthane/polyurée est
a) couplé par covalence au polymère polyuréthane/polyurée ou
b) non couplé par covalence au polymère polyuréthane/polyurée ; et piégé au sein d'une pluralité de polymères polyuréthane/polyurée.

9. Système capteur d'analyte selon la revendication 7, dans lequel le composé de stabilisation de polymère polyuréthane/polyurée présente une activité antioxydante.

10. Système capteur d'analyte selon la revendication 7, dans lequel le composé de stabilisation de polymère polyuréthane/polyurée
a) comprend au moins deux cycles benzyle ayant au moins un fragment hydroxyle ou
b) est un composé tel que défini dans la revendication 4.

11. Système capteur d'analyte selon la revendication 7, dans lequel le polymère polyuréthane/polyurée comprend :
45 à 55 % molaires de diisocyanate ;
10 à 20 % molaires de siloxane ;
30 à 45 % molaires de diol hydrophile ou de diamine hydrophile ; et
0,1 à 5 % en poids d'un composé de stabilisation de polymère polyuréthane/polyurée.

12. Système capteur d'analyte selon la revendication 7, dans lequel le composé de stabilisation de polymère polyuréthane/polyurée comprend un cycle benzyle ayant au moins deux fragments hydroxyle.

13. Système capteur d'analyte selon la revendication 7, comprenant en outre au moins l'une parmi :
une membrane de rejet d'interférences ;
une couche de protéines ;
une couche de promotion d'adhérence disposée sur la couche de détection d'analyte, dans lequel la couche de promotion d'adhérence promeut l'adhésion entre la couche de détection d'analyte et la couche de modulation d'analyte ; ou
une couche de couverture dans lequel la couche de couverture comprend une ouverture positionnée sur la couche de couverture de façon à permettre à un analyte présent dans le mammifère de venir en contact et de diffuser à travers une couche de modulation d'analyte ; et de venir en contact avec la couche de détection d'analyte.

14. Système capteur d'analyte selon la revendication 7, comprenant en outre une plate-forme de sonde et dans lequel la première sonde comprend :
un premier réseau d'électrodes comprenant une électrode de travail, une contre-électrode et une électrode de référence ; et
un deuxième réseau d'électrodes comprenant une électrode de travail, une contre-électrode et une électrode de référence ;
une deuxième sonde couplée à la plate-forme de sonde et conçue pour être insérée in vivo, dans lequel la deuxième sonde comprend :
un troisième réseau d'électrodes comprenant une électrode de travail, une contre-électrode et une électrode de référence ; et
un quatrième réseau d'électrodes comprenant une électrode de travail, une contre-électrode et une électrode de référence ;
dans lequel les premier, deuxième, troisième et quatrième réseaux d'électrodes sont configurés pour être électroniquement indépendants les uns des autres.

15. Utilisation d'une composition de matière comprenant un polymère formé à partir d'un mélange comprenant :
un diisocyanate ;
un siloxane ;
un diol hydrophile ou une diamine hydrophile ; et
un composé de stabilisation de polymère, dans laquelle le composé de stabilisation de polymère :
a une masse moléculaire inférieure à 1000 g/mol ;
comprend un cycle benzyle ayant au moins un fragment hydroxyle (ArOH), pour la fabrication d'un dispositif pour la détection d'un analyte.
